# EUROPEAN PATENT APPLICATION

(11) **EP 0 522 882 A2**
(43) Date of publication of application: **13.01.1993**
(21) Application number: 92306389.5
(22) Date of filing: 13.07.1992
(51) Int. Cl.: A43B 3/00

(54) **Products incorporating piezoelectric material**

(30) Priority: 12.07.1991 GB 9115196
(71) Applicant: Mott, Jonathan Christopher, Bentley, Farnham, Surrey GU10 5EU (GB)
(72) Inventor: Mott, Jonathan Christopher, Bentley, Farnham, Surrey GU10 5EU (GB)
(74) Representative: Shackleton, Nicola

(57) **Abstract**

A product, in particular, a shoe, apparel, a ball or a fishing lure, incorporating an impact sensing element (218) made from polymeric piezoelectric material. In response to impact, the piezoelectric material generates an electrical signal to a battery powered light or sound-emitting unit (216) or to an information display device which is at least partially molded into or contained in the product (210), thus causing circuitry to energize the light or sound-emitting device (216) from the battery or to display information on the information display device.

## Description

### FIELD OF THE INVENTION

This invention relates to products incorporating piezoelectric material, and is more particularly but not exclusively concerned with such products in the form of sports shoes, balls and fishing lures.

### PRIOR ART

It has already been proposed, for example in U.S. Patent No. 4,748,360 to Taylor, to provide dancing shoes and a child's ball each incorporating a layer of piezoelectric material and a light source. The piezoelectric material and the light source are directly electrically connected together so that the electrical energy produced by the piezoelectric effect upon impact of the shoe or ball against the ground or other surface, directly energizes the light source.

However, the amount of electric energy normally produced in response to a typical impact is insufficient to produce readily visible energization of the light source, i.e. energization of the light source which is clearly visible in daylight and/or of significant duration.

Numerous U.S. references describe lighted shoes, shoes incorporating piezoelectric materials, other articles incorporating piezoelectric materials and pressure sensitive articles, see, for example the following:
U.S. Patent No. 1,597,823 to Randolph discloses a shoe having an illuminating means disposed within the heel thereof. The illumination means is controlled by a manual switch mechanism. U.S. Patent No. 3,239,696 to Burkhalter, et al. discloses a piezoelectric pressure transducer that responds to pressure variations ranging from subaudio frequencies into, at least, the audio range. The transducer is used in sphygmomanometry to measure the systolic and the diastolic limits of blood pressure variation and for other medical applications.

U.S. Patent No. 3,323,367 to Searle discloses a grip indicator for measuring the pressure exerted by a player on a club or racquet. The grip indicator comprises one or more sensors whose electrical resistance changes when compressed and a meter and bridge circuit to measure this change.

U.S. Patent No. 3,549,878 to Bailey discloses a light distribution system for distributing a pattern of light over the clothing of a wearer. In a typical embodiment, plunger-type switches are mounted in various locations in the shoes of a wearer. Pressure imparted to various parts of the shoes, as in dancing, activates these switches. These switches, in turn, activate corresponding switches which control the amount of current applied to a piezoelectric spiral. Variations in the current applied to this spiral cause the spiral length to increase thereby rotating the tip of the spiral. This rotational motion of the spiral tip is used to control the rotation of a multicolor light filter and thus change the pattern of light produced.

U.S. Patent No. 3,582,691 to Sonderegger, et al. discloses a force transducer unit comprising piezoelectric elements mounted so as to divide the external forces applied into exactly defined partial forces.

U.S. Patent.No. 3,582,692 to Palini discloses an improved piezoelectric transducer responsive to changes in pressure and a circuit for activating a reed relay. The transducer includes a piezoelectric element, a pair of electrodes on opposite sides of the element, means for supporting the element, and an actuator means connected to the element for applying pressure to the element.

U.S. Patent No. 3,604,958 to Palini discloses a transducer for use in a security device to protect objects from unauthorized removal. The device senses both increases and decreases in pressure and provides an output signal indicating pressure changes. The transducer includes a piezoelectric element and an actuator for transmitting pressure to the piezoelectric element. When either an increase or decrease in pressure occurs, the piezoelectric element produces an output signal. This output signal decreases the conductivity of a unidirectional current device which then activates a warning circuit.

U.S. Patent No. 3,750,127 to Ayers, et al. discloses a piezoelectric sensor used as a strain gauge, an intrusion detector, or a thermal gradient detector. The piezoelectric sensor produces an electrical signal proportional to the amount of strain or deformation sensed.

U.S. Patent No. 3,798,474 to Cassand, et al. discloses a pressure wave sensor having a piezoelectric element with two faces. Each face is associated with a flexible conducting electrode over the entire length of the sensitive element. The sensor may be used for measuring pressure variations resulting from seismic vibrations in the sea or on the earth.

U.S. Patent No. 3,893,247 and 3,946,505 to Dana, III disclose shoes having illuminated sole and heel portions. A battery circuit and lamp source are located in the heel and/or sole of the shoe. The lamp is turned on and off by a tilt switch and/or a manually actuated switch.

U.S. Patent No. 3,974,491 to Sipe discloses a device for activating a buzzer when a predetermined load is placed on one's foot. The device comprises a resilient liquid-filled tube mounted within a foot pad, the tube extending from adjacent the toe end of the foot pad to adjacent the heel end. Pressure on the liquid compresses a spring which at a predetermined pressure causes the buzzer to activate.

U.S. Patent No. 4,020,572 to Chiaramonte, Jr. discloses a shoe platform having an illuminated sole. A lamp circuit and battery are located in the platform and controlled by a manual switch.

U.S. Patent No. 4,054,808 to Tanaka discloses a vibration detecting device for use with musical instruments for detecting vibrations therein. The device comprises a structure containing a piezoelectric ceramic plate housed in a case.

U.S. Patent No. 4,112,601 to Chiaramonte, Jr. discloses a shoe having a high-intensity lamp, a battery, a light modulator and filters for emitting a sequence of colored light from the lamp. The colored light is emitted through a light transmitting material comprising the riser of the shoe.

U.S. Patent No. 4,128,861 to Pelengaris discloses an illuminated shoe having a lamp and battery circuit disposed within the heel of the shoe and a means for activating the lamp comprising contacts on the heel of the shoe. Upon application of pressure to the heel, these contacts are pressed together, the battery circuit closes and causes the lamp to light.

U.S. Patent No. 4,130,951 to Powell discloses an illuminated dancing shoe having an flashlight mounted in the heel and a bundle of optical fibers which extend from the flashlight through the sole to various points around the edge of the sole. The flashlight is turned on and off by a spring operated pressure switch. This switch turns on the flashlight when the wearer applies pressure to the heel of the shoe such as by resting or tapping the heel on the dance floor.

U.S. Patent No. 4,158,117 to Quilliam, et al. discloses a pressure sensitive switch that includes a polyvinylidene fluoride sheet polarized to render it piezoelectric and a manual push button. Pressing the push button compresses the polyvinylidene fluoride sheet which then generates an electrical signal.

U.S. Patent No. 4,158,922 to Dana, III discloses a lighted shoe wherein a built-in solid state oscillator (or a tilt switch) and battery circuit flashes a lamp so as to light the transparent portion.

U.S. Patent No. 4,216,403 to Krempl, et al. discloses a transducer having a piezoelectric measuring sensor element for measurement of mechanical values on hollow bodies, especially pressure distribution within pipes.

U.S. Patent No. 4,253,253 to McCormick discloses an ornamental shoe having a transparent heel with a battery circuit and a lamp. When the lamp is turned on, it lights the transparent heel. U. S. Patent No. 4,304,126 to Yelke discloses a transducer for sensing and monitoring the injection of fuel into a fuel injection engine. The transducer includes a piezoelectric element mounted on the fuel line to sense the change in the circumferential dimension of the line due to pressure surges in the line.

U.S. Patent No. 4,328,441 to Kroeger, Jr., et al. discloses a pressure sensitive apparatus for producing electrical signals on two electrodes. The apparatus includes a conductive layer, a piezoelectric polymer film layer, an electrode layer, an insulating layer, another electrode layer, another piezoelectric polymer film layer and another conductive film layer. This reference teaches the use of both high impedance voltage sensing interface circuits and low impedance current sensing interface circuits to process the signal output by a piezoelectric sensor.

U.S. Patent No. 4,402,147 to Wu discloses an electronic counter mounted in the toe of a shoe which counts the number of steps taken. Contact switches that close when walking a step activate counter circuitry which then causes a digital display to either count up or count down.

U.S. Patent No. 4,499,394 to Koal discloses a piezoelectric sensor attached to the foot of an animal to continuously measure the pressure of the animal's foot against a reactive surface. The sensor and associated circuitry is electrically interconnected with telemetry and analyzing apparatus.

U.S. Patent No. 4,510,704 to Johnson discloses a mechanical or electronic pedometer which is contained in the heel of a shoe. The pedometer detects when a step is made and counts and displays the total number of steps taken. In the electronic counting embodiment, the pedometer includes electronic counting and processing circuitry, a battery, an impulse transducer (i.e. a piezoelectric element) to trigger a count, and an electronic display of the total count.

U.S. Patent No. 4,660,305 to Medler, et al discloses a tap dance shoe wherein impact sensors including piezoelectric transducers are contained in the taps attached to the dance shoe. The sensor produces an electrical signal when the tap strikes the dance floor. This electrical signal is then transmitted for remote processing and amplification.

U.S. Patent No. 4,703,217 to Ratzlaff, et al discloses a piezoelectric transducer mounted on a horseshoe to enable measurement of the forces generated when a horse's hoof contacts the ground. The signal from the piezoelectric transducer is amplified and processed by electronic circuitry so that its information content can be stored and used in scientific studies of a horse's locomotion pattern.

U.S. Patent No. 4,748,366 to Taylor discloses novel uses of piezoelectric materials for creating optical effects which are directly powered by the electrical energy generated by the piezoelectric materials. The piezoelectric material is incorporated into an article of manufacture such that by using the article, mechanical energy is imported to the piezoelectric material, thus causing the generation of an electrical voltage. The voltage is transferred to an optical effect device which can be activated by the high voltage, low current output of the piezoelectric material. Such devices include electroluminescent materials, gas discharge (plasma) panels, neon bulbs and liquid crystal devices. The output of the piezoelectric material is directly coupled to the optical effect device. This reference also teaches the use of layers of piezoelectric material to generate the energy required to activate the optical effect.

U.S. Patent No. 4,771,394 to Cavanagh discloses a pair of running shoes provided with a housing at the heels in which an electronic footstrike counting device may be removably mounted thereon. The electronic device is cabled to a computer before and/or after usage of the shoes to transfer footstrike count and run time data to the computer or to have the computer preprogram the electronic device with distance data so that when a given distance is completed, a tone will sound. The electronic device includes an inertia switch for producing a foot strike count. U.S. Patent No. 4,814,661 to Ratzlaff, et al. discloses a system for measuring and analyzing the forces exerted during running or walking. The system includes piezoelectric sensor elements mounted on a plate which may or may not be incorporated into a shoe. The plate extends under all or portions of the plantar surfaces of a human foot to provide accurate foot force detection during unrestrained motion.

U.S. Patent No. 4,824,107 to French discloses a martial arts sports scoring device wherein piezoelectric film is mounted on protective equipment such as headgear, handgear or footgear, protective vests and the like. The piezoelectric film produces an electrical signal with an amplitude corresponding to the force of a blow or the degree of deformation of the film. The electrical signal can be processed by electronic circuitry to count the number of blows or to measure the amount of force of each blow. The results of this analysis can be displayed on various types of displays including meters or a bar of light emitting diodes, or these results can be converted into audible tones.

U.S. Patent No. 4,848,009 to Rogers discloses footwear having a motion responsive switch, a battery, a timing circuit, and a lamp, preferably a light emitting diode. This reference teaches using a motion responsive mercury switch that alternates states in response to the motion of the footwear. When the motion causes the switch to activate (ON), a timing circuit is activated and the lamp is lit. After a predetermined time, the timing circuit turns off the lamp and prevents the lamp from being turned on again until the motion responsive switch makes an OFF transition and then an ON transition. Thus this reference teaches turning a lamp ON again only in response to a change in orientation of the footwear after the timing circuit times out. This reference does not teach lighting a lamp in response to pressure or impact on a sensor embedded in an athletic shoe.

U.S. Patent No. 4,991,150 to Wixom discloses a dynamic mechanical stress transducer comprising a piezoelectric material in intimate electrical communication with an electroluminescent material that emits light at an intensity in proportion to the magnitude and rate of change of the stress applied to the piezoelectric material. In a preferred embodiment, the electroluminescent material comprises a light emitting diode.

References uncovered relating to toys, for example balls, are:
U.S. Patent No. 3,580,575 to Speeth discloses an impact toy, such as a ball, with a circuit, impact operated switches and three colored lamps mounted in the interior. Upon impact of the toy, one of the three switches closes and a corresponding lamp lights.

U.S. Patent No. 3,610,916 to Meehan discloses an illuminable ball with an inertia switch and a time delay circuit. The ball is translucent and within the ball is an inertia switch which triggers a time delay circuit that then applies battery power to two lamps. Power is applied for a duration preselected by the time delay circuit.

U.S. Patent No. 4,595,200 to Shishido discloses a sound emitting ball. In one embodiment, the external impact force is detected by a piezoelectric sensor which triggers electronic processing and amplification circuitry. This circuitry then causes a piezoelectric speaker to emit a buzzer sound or a melody sound.

U.S. Patent No. 4,737,134 to Rumsey discloses a ball that produces audible tunes corresponding to the amount of light that falls upon a light transducer mounted on the surface of the ball. An oscillator is coupled to the light transducer and to a speaker to produce the tones. A motion switch turns off the audible tone when the ball is at rest for a preset time period.

References uncovered relating to fishing lures are:
U.S. Patent No. 3,828,177 to Day discloses a fishing lure having a battery and a lamp disposed therein. A bundle of optical fibers extends from the lamp to the exterior of the lure to conduct light to the outside of the lure.

U.S. Patent No. 3,940,868 to Northcutt discloses a fishing lure having a battery and a light emitting diode that is turned on by a water activated switch.

U.S. Patent No. 4,250,650 and 4,347,681 to Fima discloses a fishing lure containing one or more lamps and a guideway along which a battery rolls as the lure moves. Movement of the battery along the guideway intermittently completes a lamp circuit and turns on the lamps. The light from these lamps is transmitted by optical fibers to the surface of the lure.

U.S. Patent No. 4,741,120 to Cota, et al discloses a self-illuminating fishing lure having a constant light source produced by a tritium capsule encapsulated within a hard translucent fish lure body.

Foreign references uncovered relating to this invention are:
Italian Patent No. 489,219 to Valentino discloses a shoe with an on/off switch and a lamp in the heel which illuminates the heel.

French Patent No. 1,555,306 to Deus, et al. discloses an illuminated shoe having a piezoelectric transducer, which when compressed, lights a lamp.

French Patent No. 2,227,714 to Rich discloses a shoe with an illuminated heel having a battery, a mercury switch, a relay and a lamp arranged within the heel so that when the shoe is in a flat position, the lamp is turned off.

French Patent No. 2,556,190 to Hume discloses a shoe having a battery powered flashing lamp in the heel. Lamp current passing through a bimetallic switch heats the switch thereby opening the lamp circuit; the bimetallic switch then cools and closes the lamp circuit, thus causing flashing of the lamp.

Netherlands Patent No. 8006456 to Maria De Nijs, et al., discloses a battery circuit, a switch, a lamp or light emitting diode, a colored bead, and an optical fiber interconnecting the lamp with the colored bead.

Japanese Application No. 58-195238 to Daitou discloses an electro-mechanical transducer comprising a high polymer film having localized piezoelectric properties. This transducer may be used as a contactless keyboard.

European Patent Application No. 83 307822.3 to Dana III discloses a shoe having a lamp or light emitting diode, a battery circuit and a mercury switch or a mechanically activated pressure switch to light or flash the lamp. Flasher circuitry can be added which is manually activated by the wearer. In addition, the flasher circuits and battery can be encapsulated.

German Patent No. 26 08 485 to Ben-Hassine discloses an illuminated shoe heel having a battery circuit and a mercury switch for turning on and off a plurality of lamps or light emitting diodes mounted through the surface of the shoe heel. The mercury switch turns off the lamps when the heel is raised.

International Application No. PCT/AU86/00324 to Hopper discloses a touch or proximity switch that incorporates a light emitting diode to visually indicate the on or off state of the switch. A piezoelectric stress sensor may be used to trigger circuitry that lights the light emitting diode and to trigger a solid state switch or a transistor.

PCT/US80/01677 to Kroeger, Jr., et al. discloses a pressure sensitive apparatus for producing electrical signals on two electrodes. The apparatus comprises a layered structure including a conductive layer, a piezoelectric polymer film layer, an electrode layer, an insulating layer, another electrode layer, another piezoelectric polymer film layer and another conductive layer. Kroeger teaches the use of a high-impedance interface such as a digital gate or an operational amplifier circuit to sense the voltage generated by the pressure sensitive piezoelectric sensor.

Other references uncovered are:
Biomechanics VII-B, International Series on Biomechanics, Volume 4B, Proceedings of the Eighth International Congress of Biomechanics, Nagoya, Japan, 1983, discloses a shoe having an array of piezoelectric transducers embedded in silicone rubber and inserted into the mid-sole of a shoe. These transducers are used to obtain force measurements for studies on the interaction between the foot and the shoe while walking. The device further includes a back pack containing amplification and multiplexing circuitry. Medical and Biological Engineering and Computing, Foot-Force Measuring Device for Clinical Assessment of Pathological Gait, Miyazaki, et al., July 1978, discloses a force transducer that is attachable to a shoe, in combination with an amplifier transmitter and a receiver processor unit for measuring the static and dynamic forces acting between the foot and the floor during walking. The Complete Handbook of Athletic Footwear, Cheskin, Melvyn P., Fairchild Publications, New York, 1987, P. 158, discloses a flashing safety light component for use with running shoes. The device is attachable, by cement, to the heels of running shoes and functions as a night-running safety device.

None of the foregoing references teach or suggest the invention claimed herein.

### OBJECTS AND SUMMARY OF THE INVENTION

According to one aspect of the present invention, there is provided a product, preferably an athletic shoe or athletic apparel, adapted to emit light, sound energy or information in response to impact. The product comprises a molded part having a battery-powered light-or sound-emitting unit or a liquid crystal device at least partially molded therein. The unit comprises an impact-sensing element made from a polymeric piezoelectric material, a battery means, a light- or sound-emitting device or an information display device and a circuit connected to said piezoelectric material. The unit is responsive to electrical energy produced upon impact which permits the light or sound-emitting device to be energized from the battery or any information displaying device to be activated.

In one embodiment, the electrical energy resulting from each impact is used as a trigger to operate the light- or sound-emitting unit via the circuit incorporated therein, and the amount and/or duration of the light or sound emission can be independently determined/controlled by appropriate design of the circuit.

The circuit preferably comprises a monostable circuit such as a monostable multivibrator or the like, the time constant of which determines the duration of energization of the light- or sound-emitting device and may advantageously be adjustable, for example by means of a variable resistance.

Where the product includes a light-emitting device, the device may be a light-emitting diode ("LED") or a gas discharge lamp such as a neon lamp. Additionally, the light-emitting device may be disposed within the product, and the product may further include optical fiber means arranged to conduct light emitted by said light-emitting device to an outside surface of the product. Where the product contains an information display device, a liquid crystal device may be used.

The product is preferably a shoe, particularly a running shoe or a jogging shoe. The molded part is preferably a unitary sole-and-heel structure, with at least the battery, the circuit and the light-emitting device disposed in or molded into the heel part of the structure, and with the light-emitting device being arranged to emit light rearwardly from said heel part. The polymeric piezoelectric material may be molded into the sole part of the structure, preferably in the region of maximum stress.

Another embodiment of a shoe may include a plurality of light-emitting devices, either positioned at a plurality of points over the outside upper surface of the shoe or connected to said plurality of points via respective optical fibers. These light emitting devices are selectively energizable in a predetermined sequence, for example in response to successive groups or groupings of a predetermined number of impacts sensed by the impact-sensing element.

In still another embodiment, the circuit responds to the magnitude of the electrical energy produced by the piezoelectric material and thereby selectively energizes one or more of the light emitting devices depending on the amount of electrical energy produced. In this manner, a visual indication of the magnitude of the pressure exerted upon the sole of the shoe can be displayed.

In another embodiment, the light emitting devices may also be arranged to form a bar graph display. This embodiment is particularly suitable for use as an exercise shoe in performing sequences of different exercises, since different light-emitting devices, perhaps differently colored, can be energized for each different exercise.

According to another aspect of the invention, the shoe is provided with a plurality of light-emitting devices, and with temperature sensing means responsive to the temperature within the shoe in addition to or instead of the piezoelectric material. In this embodiment, the circuit, additionally or alternatively, responds to the temperature sensing means so as to selectively energize the light-emitting devices and provide a display indicative of the temperature within the shoe. Thus the light-emitting devices may be of different colors, a first color, for example green, representing a normal temperature, a second color, for example orange, representing a somewhat higher and/or lower temperature, and a third color, for example red, representing an even higher and/or lower temperature, the devices being arranged as a linear array.

In yet another embodiment, the circuit, additionally or alternatively, energizes at least one light-emitting device, independent of the piezoelectric material, at a preset or a programmable rate or rates so as to provide a pace-setting facility to the wearer of the shoe. Advantageously, the circuit also responds to the electrical energy produced by the piezoelectric material to modify the programmable rate or rates so as to tend to assist the wearer of the shoe to compensate for any difference in actual pace as compared to the initially programmed pace rate or rates.

Alternatively, a shoe with a plurality of light-emitting devices can also incorporate a plurality of impact-sensing elements distributed throughout the sole of the shoe; the circuit energizing the light-emitting devices in accordance with the relative stress sensed by each impact-sensing element so as to provide an indication of the weight distribution over the sole of the shoe or the suspension, compression, cushioning efficiency energy return and/or impact of the shoe on the surface. This embodiment is particularly suitable for use as an athletic, gymnastic or dancing training shoe, since it can provide useful information on the style or angle of foot-ground contact of an athlete, gymnast or dancer wearing it.

In another embodiment, a shoe containing one or more impact sensing elements which may be distributed throughout the sole of the shoe, a battery, information processing circuitry connected to the impact sensing element or elements responds to impact by displaying appropriate information on an information display device such as a liquid crystal display (LCD).

According to another aspect of the present invention, a shoe containing an impact-sensing element made from polymeric piezoelectric material, a battery, a light-emitting device, and a circuit connected to the impact-sensing element responds to impact by energizing the light-emitting device. An optical fiber means is arranged to conduct the light emitted by the light-emitting device to an outside surface of the shoe, preferably an upper surface (i.e. a surface visible when the sole of the shoe is flat on the ground).

Thus, the optical fiber means may comprise a single optical fiber at least a part of which runs around the outside upper surface of the shoe, said part having a plurality of notches therein so as to emit light at each notch. The notches may be colored, e.g. with different colored translucent inks or dyes, so that the emitted light is correspondingly colored.

Alternatively, the optical fiber means may comprise a bundle of optical fibers, each fiber conducting the emitted light to a different point in the outside upper surface of the shoe: for example, where the shoe has a tongue, at least some of the fibers may terminate in the free end of the tongue. These points may form an abstract or geometrical pattern of light, or a logo or one or more alphanumeric characters constituting, for example, a trademark of the manufacturer of the shoe. The ends of the fibers at these various points may be colored, e.g. with different colored translucent inks or dyes.

The product may also be a fishing lure, wherein optical fibers conduct the emitted light to different points of the outside surface of the lure.

The product may also be a ball, for example, wherein at least its radially outer region is molded in a translucent polymeric material, and includes two light-emitting devices both arranged to be simultaneously energized by the circuit, the light emitting devices being arranged to emit light in generally opposite directions.

### BRIEF DESCRIPTION OF THE DRAWINGS

The invention will now be described, by references to the accompanying drawings, of which:
**Figure 1** shows a side view of one embodiment of the present invention wherein an athletic shoe incorporates a piezoelectric impact sensor;
**Figure 2** shows a cross-sectional view of another embodiment of the present invention wherein a ball incorporates a piezoelectric impact sensor;
**Figure 3** shows a schematic diagram which includes a monostable multivibrator circuit for incorporation into a product of the present invention, for example, the athletic shoe of **Figure 1**;
**Figure 4** shows a top schematic view of an athletic shoe of the present invention wherein the light-emitting diode is mounted in the sole in front of one end of a light-transmitting optical fiber bundle and the other end of these fibers terminate at various points on the outside upper surface of the shoe;
**Figure 5** shows a top schematic view of an alternative embodiment of an athletic shoe of the present invention wherein the light-emitting diode is mounted in the sole in front of one end of a light transmitting optical fiber bundle;
**Figure 6** shows an enlarged view of an optical fiber of the athletic shoe of **Figure 5**;
**Figure 7** shows a top schematic view of an alternative embodiment of the athletic shoe of the present invention wherein piezoelectric impact sensors are located in various locations in the sole and heel of the shoe;
**Figure 8** shows a top schematic view of an alternative embodiment of the athletic shoe of the present invention wherein a plurality of light emitting diodes are mounted in front of one end of an optical fiber bundle, and the fibers of the opposite end of the optical fiber bundle are embedded in various locations on the outside upper surface of the shoe;
**Figure 9** shows a schematic view of an embodiment of the present invention wherein the product is a fishing lure;
**Figure 10** shows a schematic which includes an alternate monostable multivibrator circuit for incorporation into the various embodiments of the present invention;
**Figure 11** is a graphical representation of the signal output of the circuit of **Figure 10** showing the response to impacts caused by walking or running;
**Figure 12** shows a schematic of another embodiment of a monostable multivibrator circuit for incorporation in a product of the present invention;
**Figure 13** shows another embodiment of an athletic shoe of the present invention wherein a plurality of light-emitting diodes are positioned on the shoe;
**Figure 14** shows a schematic for another circuit for use in connection with a product of the present invention;
**Figure 15** shows a perspective view of another shoe of the present invention;
**Figures 16** and **16A** show a combination exploded view and phantom view of the shoe of **Figure 15** showing the location of the components of the present invention;
**Figures 17** and **17A** show a partial side plan view of the shoe of **Figure 15** showing the piezoelectric impact sensor over the cushioning pad in the heel section of the shoe;
**Figures 18** and **18A** show a partial exploding view of the electronics capsule comprising the circuit and battery for use in connection with an athletic shoe of the present invention;
**Figure 19** shows a cross-sectional view of the components comprising the electronics capsule shown in **Figure 18**;
**Figure 20** shows a top plan view of the cap of the electronics capsule of **Figure 18**;
**Figure 21** shows a side plan view of the electronics capsule of **Figure 18**;
**Figure 22** is a diagram of the critical angles involved with positioning the light emitting diode in the heel of a shoe of the present invention;
**Figure 23** is a partially exploded top plan view of a section of the heel of the shoe of **Figure 15** with a portion of the illumination means shown in phantom; and
**Figure 24** shows a cross-sectional view of a portion of the heel of the shoe of **Figure 15** illustrating the location and configuration of the light chamber and light emitting diode.

### DETAILED DESCRIPTION OF THE INVENTION

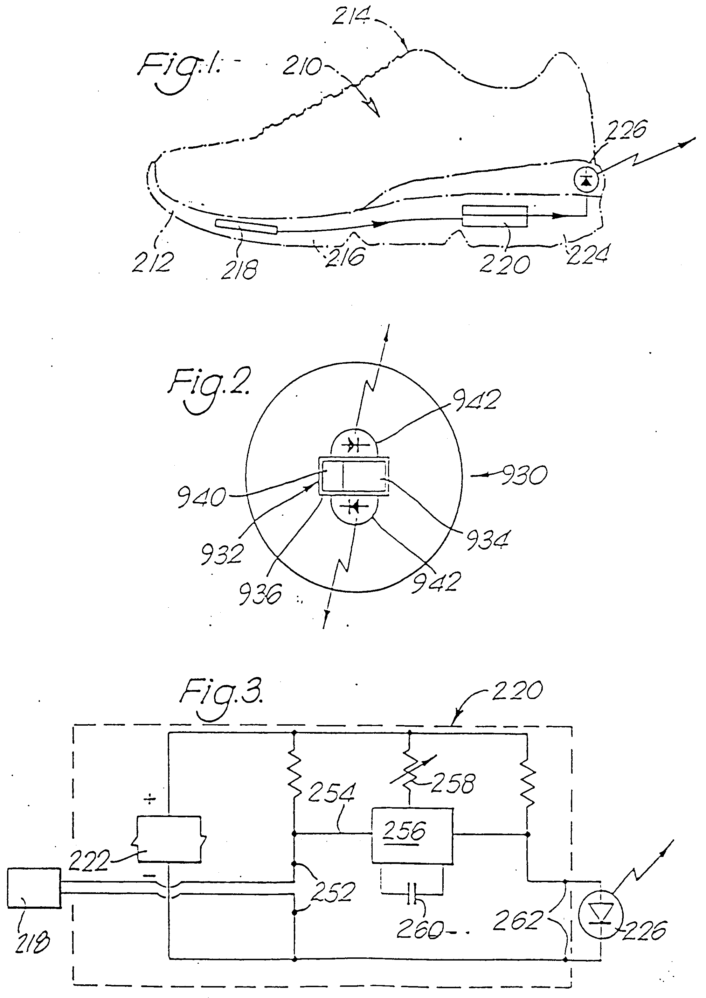

The shoe **210** shown in **Figure 1** comprises a unitary sole-and-heel structure **212** attached by molding or other means to an upper **214**. The sole-and-heel structure **212** may be molded to the upper using methods well known in the art.

Located or molded within the sole **216** of the sole-and-heel structure **212**, preferably adjacent to a point of maximum stress (i.e. near the part corresponding to the ball or heel of the wearer's foot) is a piezoelectric impact sensor **218** comprised of a sheet or layer of polymeric piezoelectric material. This piezoelectric impact sensor **218** preferably comprises polyvinylidene fluoride (PVDF) which has been stretch oriented and electrically polarized to enhance its piezoelectric properties. Such materials are known in the art. Referring to **Figures 1** and **3**, the piezoelectric impact sensor **218** is electrically connected to a circuit **220** which contains a battery pack **222**, preferably a mercury, silver oxide or lithium battery, molded into the heel **224** of the sole-and-heel structure **212**.

Referring to **Figure 3**, the circuit **220** when triggered by the piezoelectric impact sensor **218**, energizes a light-emitting diode (LED) **226**. The LED **226** may be located or molded into the heel **224** so as to emit light rearwardly therefrom.

In use, as the wearer of the shoe **210** walks, runs, jogs or moves, the piezoelectric impact sensor **218** produces a pulse of electrical energy each time the sole **216** of the sole and heel structure **212** impacts the ground, i.e. at each step or stride of the wearer, by virtue of the piezoelectric effect. Each pulse of electrical energy triggers a monostable circuit **256** such as a multivibrator circuit or the like within the circuit **220**.

Triggering the monostable circuit **256** allows the LED **226** to light for a time period determined by the resistor **258** and capacitor **260**. Resistor **258** can be variable or a fixed and could be used to preselect the amount of time that LED **226** will be lit.

In use, a bright flash of light is emitted rearwardly from the heel of the shoe **210**. Thus the wearer of a pair of the shoes **210** is clearly visible from behind by virtue of the flashes of light emitted rearwardly with each stride, which has considerable advantages from a safety point of view for runners and joggers on public roads, particularly in low light conditions.

In another embodiment, the circuitry can be molded or encapsulated with the battery or batteries and the LED. The piezoelectric impact sensor can then be attached to any surface of this encasement and then installed in a shoe such that the LED is visible from the rear of the shoe or from some point along the circumference of the sole. In an alternative version of this embodiment, the LED can be extended via wires to remote locations of the sole, heel or upper of the shoe while the encapsulated circuitry, battery, and impact sensor are located in an area of maximum impact. A plurality of these devices could be located throughout the shoe.

In yet another embodiment, of the shoe, the circuitry can be modified to operate other devices such as an electronic pedometer. In this embodiment, the circuitry reacts to impacts sensed by the piezoelectric impact sensor that exceed a certain magnitude or preset value, e.g., as when a wearer of the shoe is actually walking or running. The impacts that exceed this preset value are used to drive a digital display, thus counting the number of steps or strides taken.

**Figure 3** shows a schematic diagram for circuit **220.** The circuit **220** comprises a pair of inputs **252** electrically connected to the piezoelectric impact sensor **218** and to the control input 254 of a monostable circuit **256**. The monostable circuit **256** has a timing circuit comprising a resistor **258** (either variable or fixed) and a capacitor **260**, electrical power supply inputs connected to the battery pack **222**, and outputs **262** connected to the LED **226**. Adjustment or selection of the value of the resistor **258** determines the duration of the time period for which the monostable circuit **256** allows the LED **226** to light in response to each impact sensed by the piezoelectric impact sensor **218**.

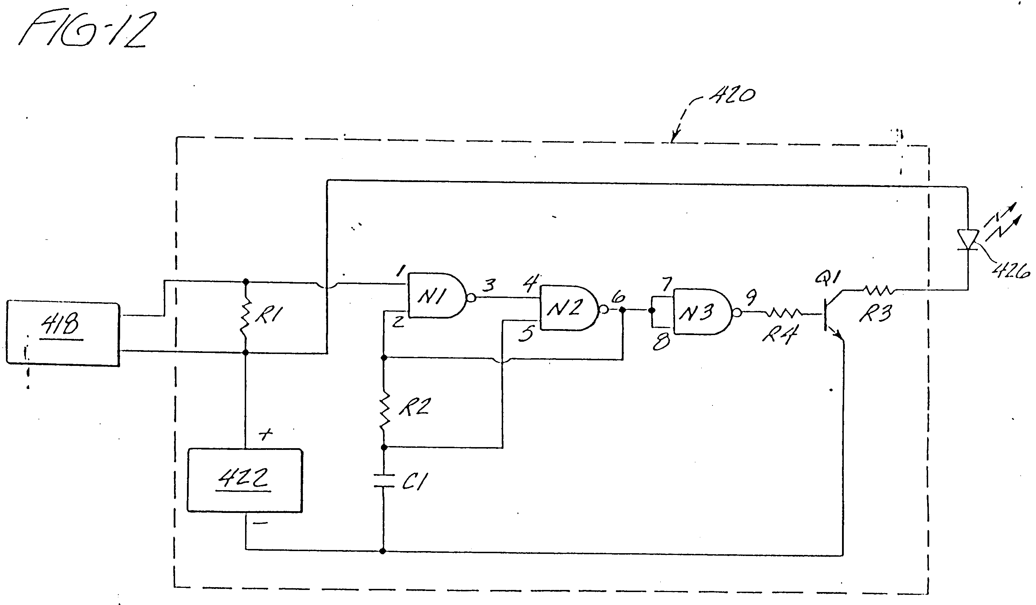

**Figure 10** shows another monostable multivibrator circuit **320** that can be incorporated in the articles of this invention. In this embodiment, the stress of an impact, such as when the shoe strikes the ground, causes a piezoelectric impact sensor **318** to generate a voltage trigger or pulse. This trigger is applied across resistor R_{S} and to the input **352** of the monostable multivibrator chip **328**. Triggering the input causes the output of chip **328** to turn on LED **326** connected to the output **356** via a current limiting resistor Rₒ and causes the capacitor C_{T} to discharge. The value of resistor Rₒ is selected to maximize the brightness of LED **326.**

After the output of chip **328** is triggered to turn on the LED **326**, capacitor C_{T} begins to charge through resistor R_{T}. When the voltage across capacitor C_{T} at input **354** reaches, for example, about two-thirds of the value of the battery voltage, the chip **328** is reset and the LED **326** is turned off. The amount of time that it takes for capacitor C_{T} to charge to two-thirds of the battery voltage is determined by the R-C time constant selected by the values used for the resistor R_{T} and capacitor C_{T}.

After the chip **328** is reset, it returns to a quiescent state. In the quiescent state, capacitor C_{T} completely charges to the value of the battery voltage and a minimum of current flows through the LED **326**. The circuit in this quiescent state thus maintains a minimum current draw so as to achieve maximum battery life.

The value selected for resistor R_{S} controls the sensitivity of the piezoelectric impact sensor **318**. Different values vary the amount of loading and thus control the magnitude of the trigger voltage applied to the chip **328**. The value selected for resistor R_{O} controls the current flow through LED **326**. This resistor value will depend on the voltage of battery **322** and the amount of current required to achieve optimum brightness of LED **326**.

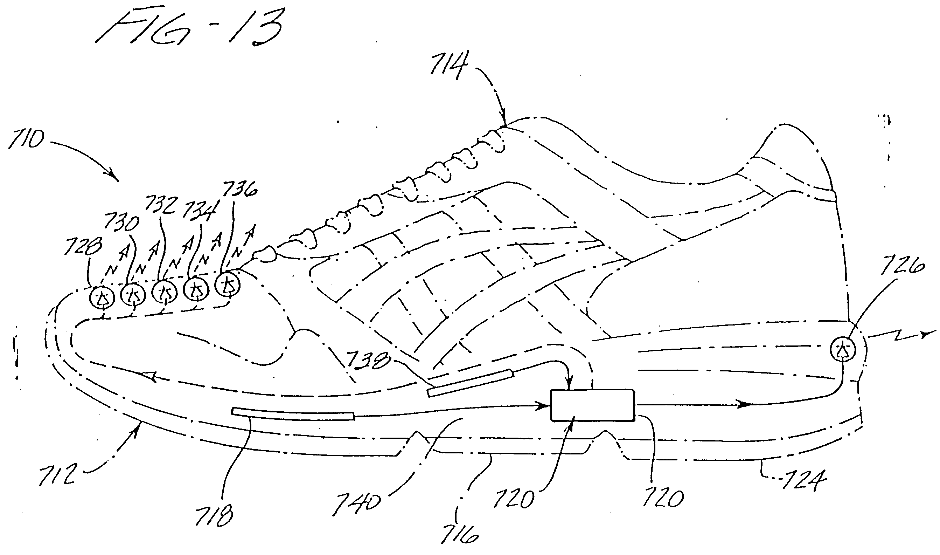

**Figure 11** shows a graphical representation of the waveforms generated by the circuitry of **Figure 10** and the on/off times of LED **326**. In the quiescent state, i.e., no impacts, the voltage applied to input **352** of chip **328** is at a minimum level **352A**. When the piezoelectric impact sensor **318** senses an impact or similar stress, it generates a voltage peak. When this peak exceeds a preselected level **352B**, as determined by the selection of an appropriate value for resistor R_{S}, then the input **352** of chip **328** is triggered and the monostable multivibrator changes state.

In the quiescent state, capacitor C_{T} is charged to the voltage of battery **322**. Thus, at this time, the input **354** is held "high" as shown by the quiescent input **354A** of **Figure 11.** Triggering the chip **328** causes capacitor C_{T} to rapidly discharge to the input "low" level **345C**. At this point, capacitor C_{T} starts to charge through timing resistor R_{T}. When the voltage across capacitor C_{T} reaches, for example about two-thirds of the battery voltage, then this input "high" voltage **354B** resets chip **328** and turns off the LED **326**.

Thus, when an input signal from the piezoelectric impact sensor **318** exceeds the trigger input level **352B**, chip **328** turns LED **326** on by outputting an output "low" level **356B**. After capacitor C_{T} recharges to the reset input "high" level **354B**, chip **328** turns LED**326** off by outputting an output high level **356A**.

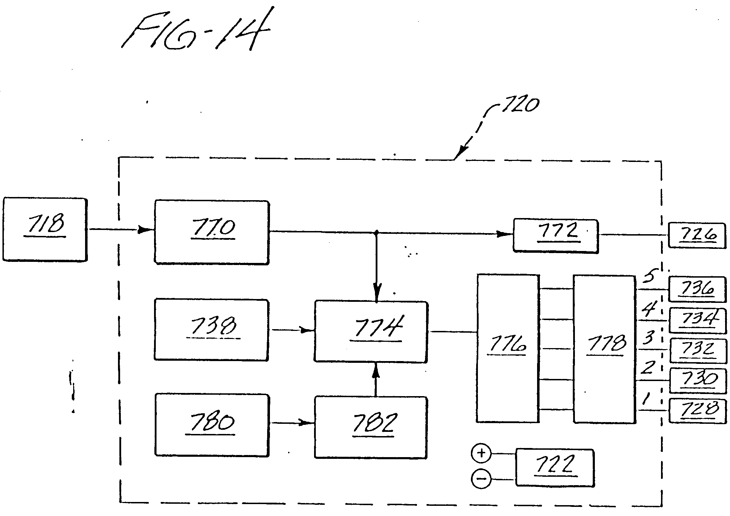

**Figure 12** shows another circuit embodiment wherein a R-S (reset-set) latch circuit **420** can be incorporated in, for example, a shoe. This embodiment has the advantages of lower cost, minimum size and very low power consumption so as to offer extended battery life. In this embodiment, an impact on the piezoelectric sensor **418** is used to trigger a R-S latch which turns on an LED **426** for a time determined by a R-C time constant.

When an impact is felt by the piezoelectric impact sensor **418** shown in **Figure 12**, such as when the shoe strikes the ground, the sensor **418** generates a small amount of current which passes through sensitivity resistor R1 and causes a decrease in the voltage or signal applied at point 1 of the R-S latch comprising NAND gates N1 and N2.

Selection of various values of sensitivity resistor R1 is used to adjust the sensitivity of the circuit **420**. Using a lower value of resistance for R1 requires a larger current flow through resistor R1 to produce a signal large enough to trigger the R-S latch; thus, decreasing the sensitivity of the circuit **420** to signals generated by the impact sensor **418**. Likewise, a higher value of resistance used for R1, increases the sensitivity since a larger signal is produced for a given current generated by the impact sensor **418**.

Thus, when the impact sensor **418** senses an impact, the signal it generates causes the input voltage applied to point 1 of NAND gate N1 of the R-S latch to go low. The output of NAND gate N1 then goes high which causes the output of NAND gate N2 of the R-S latch to go low.

The low output of NAND gate N2 causes the invertor N3 output to go high and turn on transistor switch Q1. Turning on transistor switch Q1 allows current to flow through the collector branch of this transistor and thus turn on LED **426**. Thus, an impact sensed by the piezoelectric sensor **418** causes the R-S latch to set and via transistor Q1 to turn on LED **426**.

The value of the LED current limiting resistor R3 is determined by the current required to produce the optimum brightness level of LED **426**. Thus, the resistor R3 value depends on the current required, the battery voltage, and the LED **426** and transistor Q1 voltage drop when this current is flowing through the collector circuit of transistor switch Q1.

Referring to **Figure 12**, a bias resistor R4 can be inserted in the base-emitter circuit of transistor switch Q1 to limit the base-emitter current and thus conserve battery life. In another embodiment, resistor R4 can be omitted in order to conserve space but at the cost of reduced battery life. For example, if the LED **426** draws 20 to 25 mA of current and the base-emitter circuit of transistor switch Q1 draws 2 to 2.5 mA of current without a bias resistor, then the battery life can be expected to be shortened by about ten percent due to the increased current drain when transistor switch Q1 is turned on.

In addition to causing the LED **426** to turn on when the output of NAND gate N2 of the R-S latch goes low, the low output is simultaneously applied to the R-C timing circuit comprised of resistor R2 and capacitor C1. This low output causes capacitor C1 of R-C timing circuit (which had previously charged to the battery voltage) to discharge through timing resistor R2 and NAND gate N2. When the charge on capacitor C1 decreases, for example, to about one-half the value of the battery voltage; this low input signal is applied to point 5 of NAND gate N2, and causes the output of NAND gate N2 to go high again; thus resetting the R-S latch, turning off transistor switch Q1 and turning off LED **426** and allowing the R-C timing circuit to recharge to the battery voltage through NAND gate N2. In one embodiment, circuit **420** uses CMOS technology whose input threshold is about one half the power supply voltage. The use of CMOS technology provides an ideal match between the natural trigger threshold of NAND gate N2 and the trigger input generated by the discharge of capsulator C1 of the R-C timing circuit. In addition, the use of low-power CMOS devices extends battery life.

Resetting the R-S latch returns circuit **420** to a quiescent state and enables NAND gate N1 of the R-S latch to again respond to an impact trigger signal from the piezoelectric impact sensor **418**. The amount of time that the R-S latch holds the LED **426** on is determined by the R-C timing circuit of resistor R2 and capacitor C1. By varying the value of either of these components, the amount of time the R-S latch holds the LED **426** on can be increased or decreased.

While the R-S latch is set, i.e. the R-S latch is holding LED **426** on, all signals generated by impact sensor **418** are ignored by the NAND gate N1 of the R-S latch.

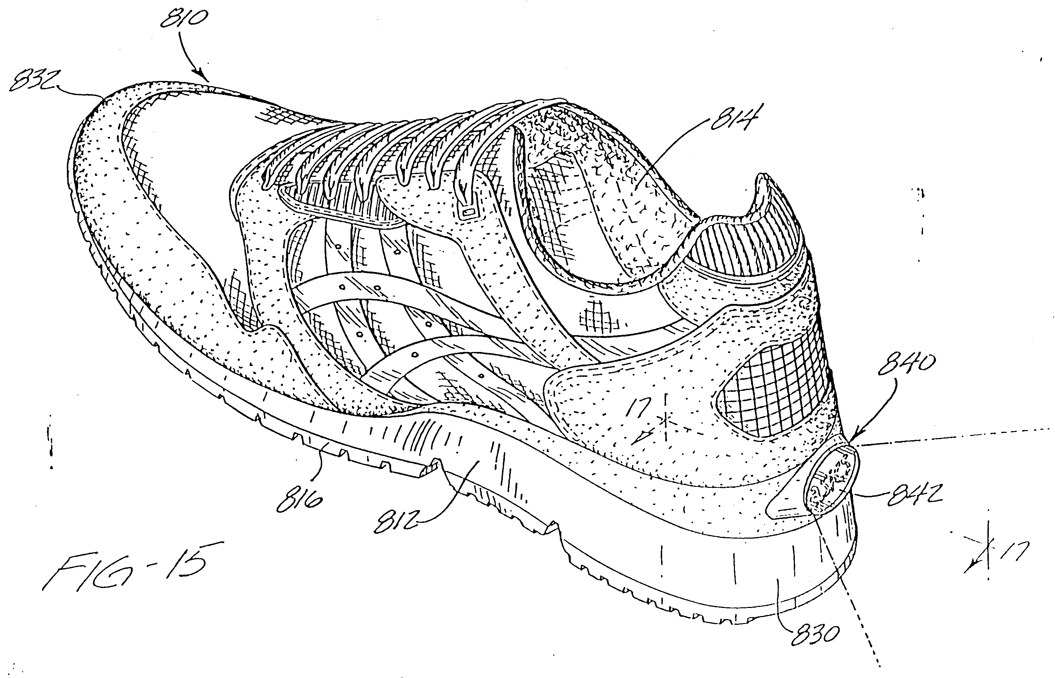

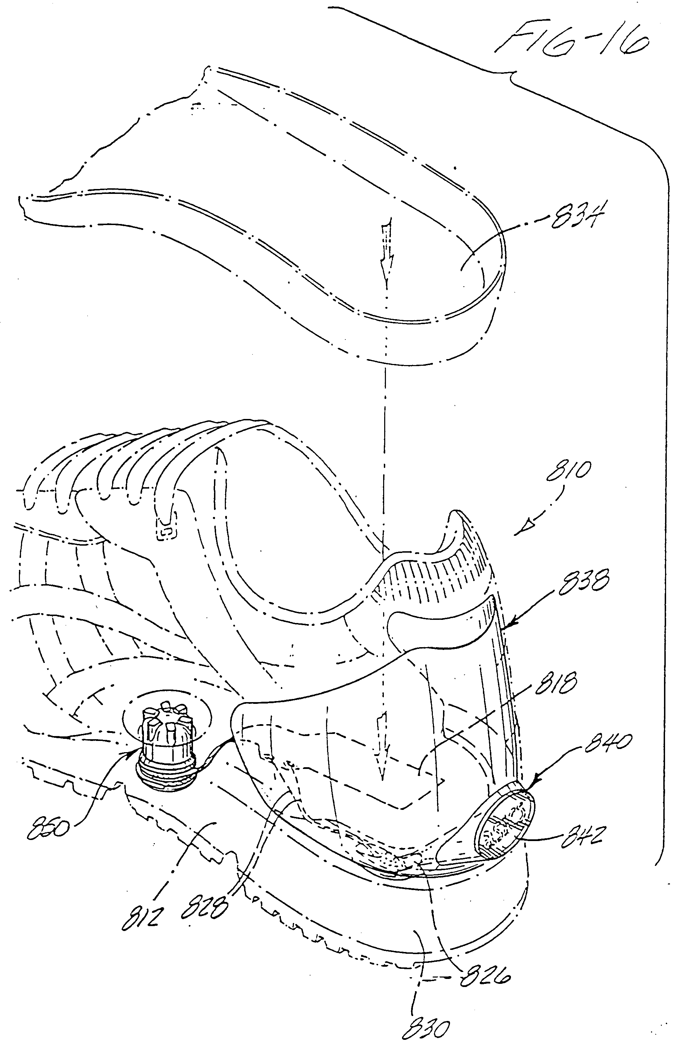

**Figure 13** depicts another embodiment of a shoe of the present invention incorporating the circuitry described herein. The shoe **710** of this embodiment is provided with numerous light emitting devices such as LEDs **728** through **736**, one or more impact sensors **718**, a temperature sensor **738** and an appropriate circuit **720** to process the impact and the temperature information. In this shoe, **Figure 14**, a circuit such as circuit **720** may be used to process impact information and use this information to turn on light emitting devices such as LEDs **728** through **736** so as to display a bar graph, or to use the magnitude of the impact to light or flash individual LEDs.

It is also within the scope of this and the other embodiments of this invention to use a circuitry responsive to temperature, which can process such temperature information so as to display temperature graphically via LEDs.

Referring to **Figure 13**, the shoe **710** incorporates within the sole and heel structure **712**, a piezoelectric impact sensor **718,** a temperature sensor **738** and a circuit **720** to process the signals received from these sensors. LEDs **728** through **736** are incorporated in the vicinity of the toe portion of the upper **714** section of the shoe **710**. These LEDs are typically positioned so as to be visible to the wearer while walking or running. Likewise, an LED **726** may be mounted in the heel **724** of the shoe **710**, and facing rearward so as to be visible to vehicles or individuals approaching from the rear.

Referring to **Figure 14,** circuit **720** of **Figure 14** illustrates a functional circuitry that may be used to process the information generated by the sensors **718** and **738** of the shoe **710** of **Figure 13** and to control the LEDs **726** through **736** incorporated in shoe **710**. Referring to **Figure 14** a microprocessor **780** can be included in circuit **720**, to provide preprogrammed control of the LEDs **728** to **736** and/or to evaluate the input from an impact sensor **718** and/or a temperature sensor **738** and then light appropriate light emitting devices (**718** to **736**). Also, a means of generating audible sounds (not shown) could be substituted for or included in addition to the light emitting devices.

Circuit **720** can process the input signals from either:
(1) the piezoelectric impact sensor **718** to light various LEDs, such as LED **726**, and optically LEDs **728** through **736**, to indicate the magnitude of the impacts suffered by the shoe; or
(2) the temperature sensor **738** to light various LEDs, such as LED's **728** through **736**, to indicate various temperature levels within the shoe.

The circuit **720** of **Figure 14 is** powered by a battery pack **722**. Circuit **720** comprises an input from piezoelectric impact sensor **718** which is applied to differentiating circuit **770** and a three state switching circuit **774** which selects one of three inputs to drive a ladder network **776**. The ladder network **776** has five outputs, each corresponding to a higher output voltage. Each of these outputs are connected via threshold circuits **778** to the appropriate LED, e.g. 1st LED, **728**; 2nd LED, **730**; 3rd LED, **732**; 4th LED, **734**; and 5th LED, **736.**

Assuming the switching circuit **774** is set to couple the output of the differentiating circuit **770** to the ladder network **776**, then the greater the impact pressure on the piezoelectric impact sensor **718**, the greater the voltage at the output of the differentiating circuit **770.** This greater voltage increases the number of active outputs of the ladder network **776** which in turn, causes the threshold circuit **778** to turn on the appropriate number of the LEDs **728, 730, 732, 734** or **736**. Thus, by lighting from one to five of these LEDs, a bar graph display of impact pressure can be seen on the toe portion. of the upper **714** section of shoe **710** (see **Figure 13**).

The switching circuit **774** can also be set to connect the temperature sensor 738 to the input of the ladder network **776**. In this case, the LEDs **728, 730, 732, 734** and **736** provide the wearer of the shoe **710** a bar graph display of the temperature adjacent the instep region of the sole **716** (see **Figure 13**). The temperature sensor **738** may be a National Semiconductor type LM35 temperature sensor, a thermistor, a temperature sensing diode, or the like, together with an amplifier where necessary. The temperature sensor can be mounted in various other locations within the shoe structure to provide a bar graph display of the temperature of the adjacent region.

As an alternative to a bar graph display of temperature, the LEDs can be of different colors, e.g., LED **728**, red; LED **730**, orange; LED **732** green; LED **734** orange; and LED **636** red, or the like, with green lit to indicate normal temperature, orange lit to indicate a higher and/or lower than normal temperature, and red lit to indicate a much higher and/or lower temperature.

The switching circuit **774** can also be set to connect the output of a programmable microprocessor **780** via a digital to analog (D/A) converter **782** to the ladder network **776**. The microprocessor **780** is preprogrammed, such as by a plug-in or a masked ROM (read-only memory) or PROM (programmable read only memory) means (not shown) or the like, to produce a series of digital pulses which are converted via the D/A converter **782** into an analog trigger signal and applied to the ladder network **776** via the switching circuit **774**. The microprocessor **780** controls the amplitude and frequency of the signal produced by the D/A converter **782**. Thus, the microprocessor **780** can cause one or more of the LEDs **728**, **730, 732, 734,** and **736** to light/flash at a rate chosen to set a desired pace or series of paces (a pacing mode) for a given distance to be walked, run or raced.

The switching circuit **774** can be manually switched between its three inputs by a manually operative switch means (not shown), or it can automatically cycle between two or all three of the various inputs, e.g. impact, temperature and pacing.

Many modifications can be made to the described embodiments of the invention. For example, in the "pacing mode", additional circuit means can be added so that the microprocessor can sense the actual pace by using the impacts sensed by the piezoelectric impact sensor. The microprocessor can then adjust the pattern displayed by the LEDs to try to compensate for any shortfall or excess between the initial set pace and the actual pace. In a further embodiment, the switching circuit **774** can cycle between the microprocessor **780** for a pace setting display and the temperature sensor **738** to provide a visual warning in the event the temperature sensed within the shoe **710** becomes excessively high.

In an alternative embodiment (not shown) similar to the embodiment of **Figures 13** and **14**, the shoe is provided with one or more input sensors, such as one or more piezoelectric impact sensors, and/or a temperature sensor, interface circuitry to connect these sensors to a microprocessor, a microprocessor to process the information and to then, via output control circuitry, to control various output devices; information display devices such as an LCD, light emitting devices such as one or more LED's or sound emitting devices. In addition to processing information from the input sensors, the microprocessor can provide preprogrammed control of the various output devices.

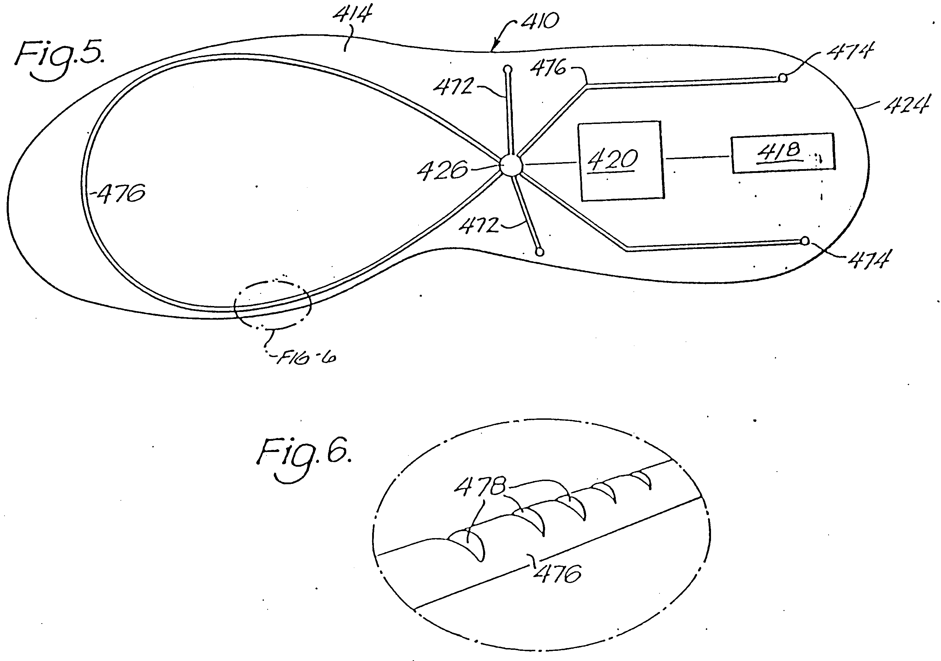

Many additional modifications can be made to the described embodiments of the invention. For example, **Figure 5** shows an athletic shoe of the present invention comprising a piezoelectric impact sensor **418**, a R-S latch circuit **420**, such as any of the circuits described herein, or the like, including a battery and an LED **426** which can be formed as a single encapsulated unit which is removably fitted into the heel **424** of the shoe. The LED **426** of this encapsulated unit is arranged to emit light rearward by transmitting the light emitted by the LED **426** via optical fibers **476** to points on the outside surface **474** of the heel **424**.

Still referring to **Figure 5,** an optical fiber **476** or other light guiding medium (e.g. a layer of suitable translucent plastic material) can be molded into the shoe **410**, so as to guide the light emitted by the LED **426** to various external surfaces of the shoe, e.g. to an exit point **474** facing rearwardly from the heel **424**, to one or more ribs **472**, extending vertically up and down the heel or to loop around the front portion of the upper **414**. This embodiment provides for ready replacement of the capsule when the battery is exhausted, or when an LED of one color is to be replaced with an LED of another color. Alternatively, just the battery can be arranged to be replaceable.

Optionally, as shown in **Figures 5** and **6**, at least a part of the optical fiber bundle can be replaced by a single optical fiber **476** which runs around the upper part **414** of the front of the shoe and which has a plurality of notches **478**, distributed along its length. From each of the notches **478** light from the LED **426** is emitted. These notches **478**, as well as the points **474** on the outside surface of the shoe can be colored, for example with different colored translucent inks or dyes, to create a variety of multi-colored effects.

Referring to **Figure 4**, another embodiment of shoe **310** has a LED **326** used in conjunction with an optical fiber bundle **370**, the individual fibers **372** can be arranged to conduct the light from the LED **326** to respective points **374** on the outside surface of the shoe, e.g. distributed in an abstract or a geometric pattern over the upper part of the front of the shoe, or distributed to form a logo or one or more alphanumeric characters constituting a trademark of the manufacturer of the shoe, or disposed throughout the free end of the tongue of the shoe.

Still referring to **Figure 4**, the piezoelectric impact sensor **318** can be mounted in the heel or other impact sensitive area. Circuit **320** comprises a monostable multivibrator circuit, or the like, and a battery. Signals from impact sensor **318** trigger circuit **320** which then turns on LED **326** for a period of time determined by a resistor-capacitor time constant incorporated into circuit **320**.

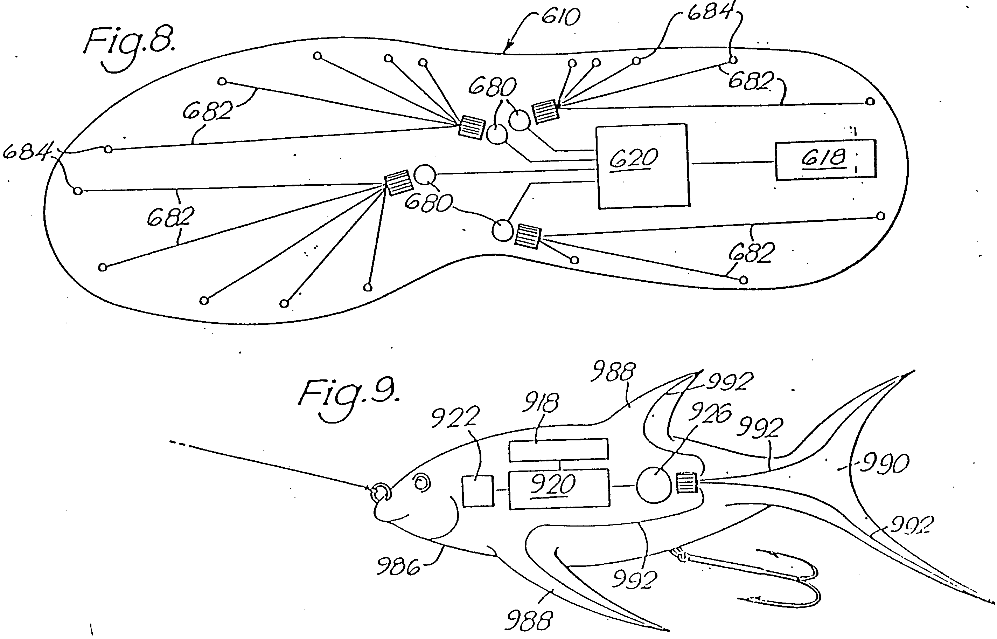

Referring to **Figure 8,** in another embodiment, shoe **610** includes a plurality of light emitting devices disposed within the shoe. The light emitted by LEDs **680** are transmitted to a plurality of points **684** on the outside surface of the shoe via optical fibers **682**. In this embodiment, piezoelectric impact sensor **618** triggers circuit **620**. Circuit **620** then turns on all LEDs **680** or alternatively, circuit **620** could be designed to selectively light or flash various LEDs **680** in a sequential, random or other pattern.

A modification to this embodiment is to incorporate circuitry such as counter and gating circuits wherein successive impacts detected by the piezoelectric impact sensor are counted. Based upon these counts, the circuitry sequentially lights successive LEDs at different locations in the shoe. For example, on the occurrence of a certain number of counts, the circuitry could light a selected LED so as to cause it to flash say ten or twenty times. Upon the occurrence of the next preselected count value, the circuitry could successively, randomly or in some preselected order, cause one or more of the other LEDs to light and/or flash for a period of time or for a set number of flashes.

This modification is particularly useful in an exercise shoe, since the pattern of lights can be used to assist and/or indicate the timing of a sequence of different exercises, with perhaps a different color LED being selected for the various exercises.

A further modification of this embodiment is to incorporate microprocessors or counting circuitry to analyze and process the signal generated by impacts detected by the piezoelectric impact sensor and then to display a count or other information based upon this information processing on an information display device such as an LCD connected to the microprocessor or counting output circuitry.

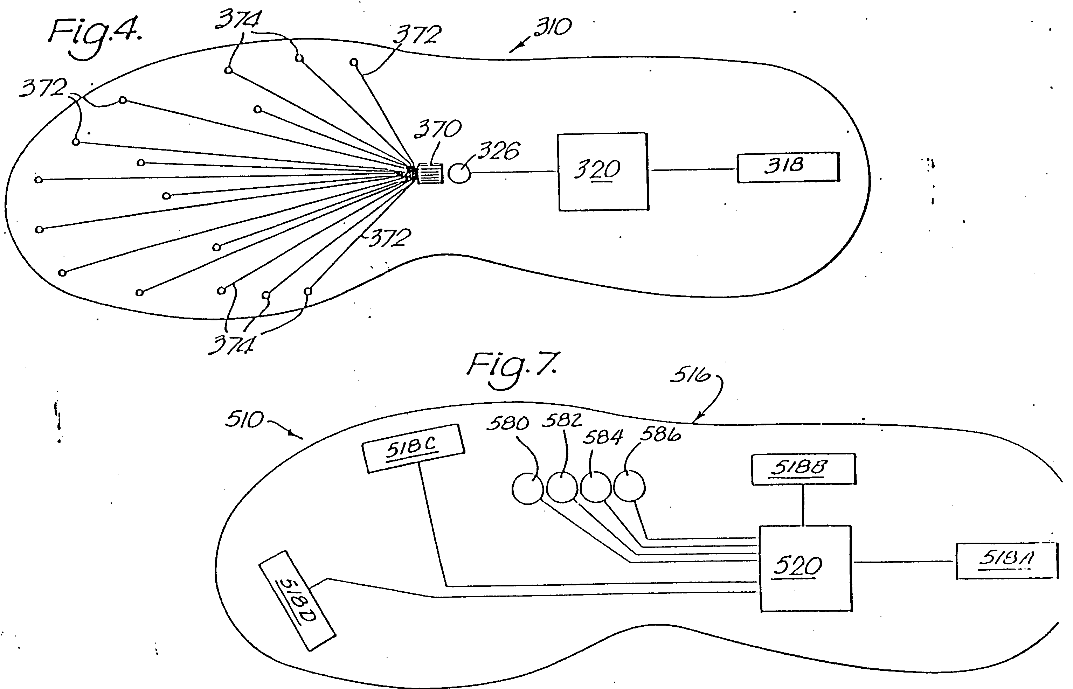

Referring to **Figure 7**, depicting still another embodiment of the present invention, in addition to a plurality of LEDs, shoe **510** may also incorporate a plurality of individual piezoelectric impact sensors **518A** through **518D** distributed throughout the sole 516 of shoe **510**. In this embodiment, a circuit **520** is designed to light one or more LEDs **580** through **586** to reflect the relative stress sensed by the piezoelectric impact sensors **518** so as to provide an indication of the weight distribution over the sole of the shoe. This forms an excellent training aid for athletics, particularly runners, as well as for gymnasts and dancers, and is very effective if the athlete, gymnast or dancer is filmed in action with a video camera and the film is then played back in slow motion.

In embodiments of the shoe involving optical fiber bundles, one or more of the LEDs can, if desired, be omitted, their effect being achieved by use of ambient light. Thus, the optical fiber bundles act as light gatherers, so that if a bundle of their "input" ends is disposed, for example, all over the front of the shoe facing generally forwardly and upwardly, while their corresponding "output" ends are disposed in the heel, facing rearwardly and distributed to form, for example, a logo or alphanumeric character constituting a trademark, ambient light will in many circumstances be sufficient to make the "output" ends of the optical fibers appear to light up. Again, multicolored effects can be created, if desired, as described earlier.

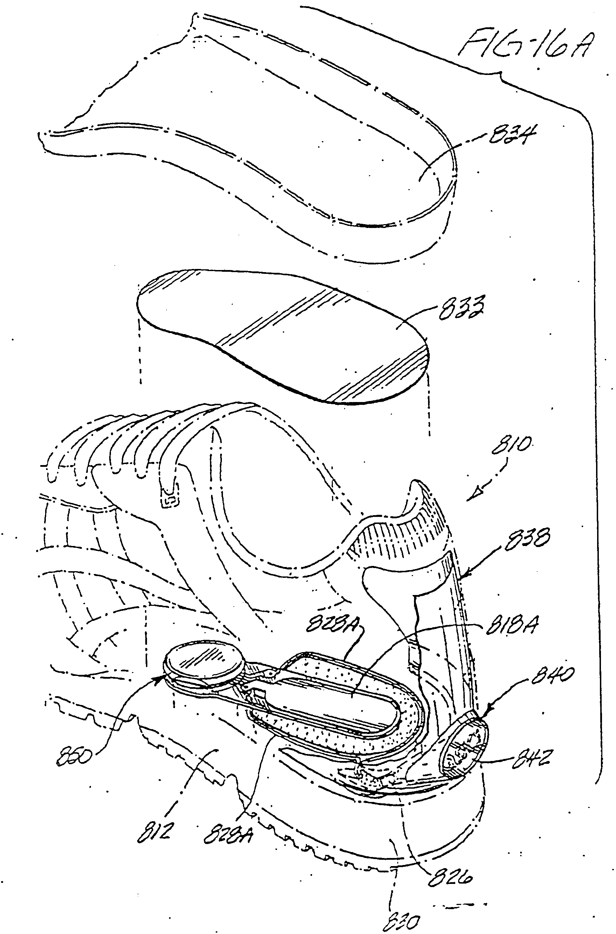

**Figures 15** through **24** depict preferred embodiments of the shoe of this invention. Referring to **Figure** **15,** shoe **810** has a light unit **840** incorporated into the proximal end **830** of the shoe **810.** The shoe **810** comprises a sole **816**, a midsole **812,** an upper **814,** a proximal (or heel) end **830,** a distal (or toe) end **832** and a light unit **840.**

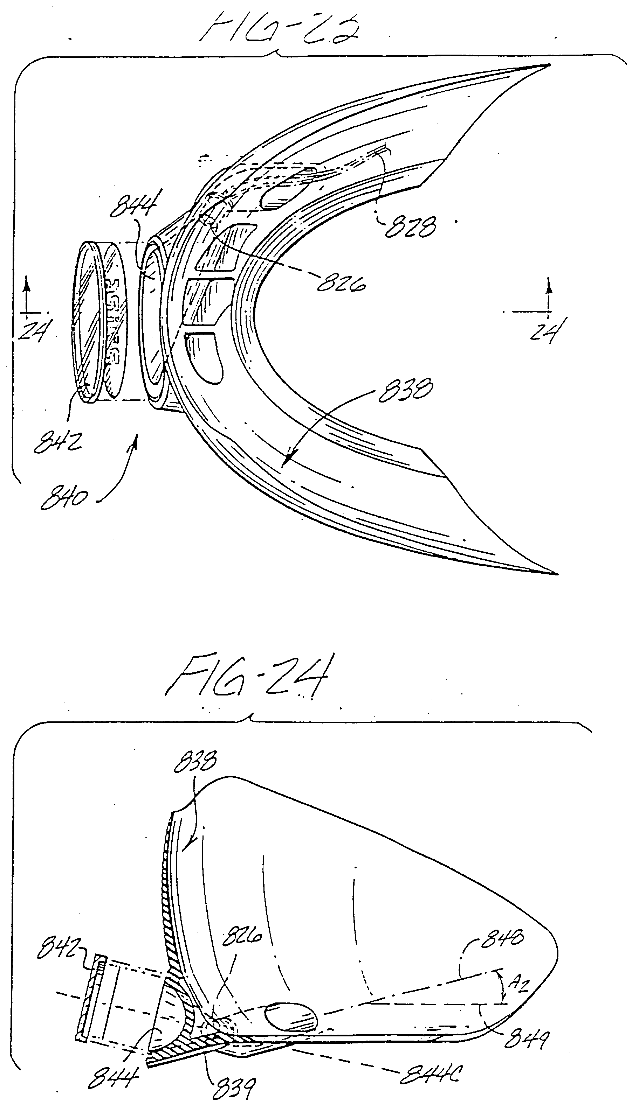

The light unit **840** comprises a lens **842** on which a name or logo may be imprinted. When an internally mounted light emitting unit such as LED **826** shown in **Figure 22**, is turned on, the logo or imprint is illuminated and is visible from the rear of the shoe.

The lens **842** can comprise translucent or colored material or a filter material so that the lens and/or imprint can be illuminated in various colors and/or textures to both improve the visibility of the shoe as viewed from the rear and to enhance the projection of any imprint thereon.

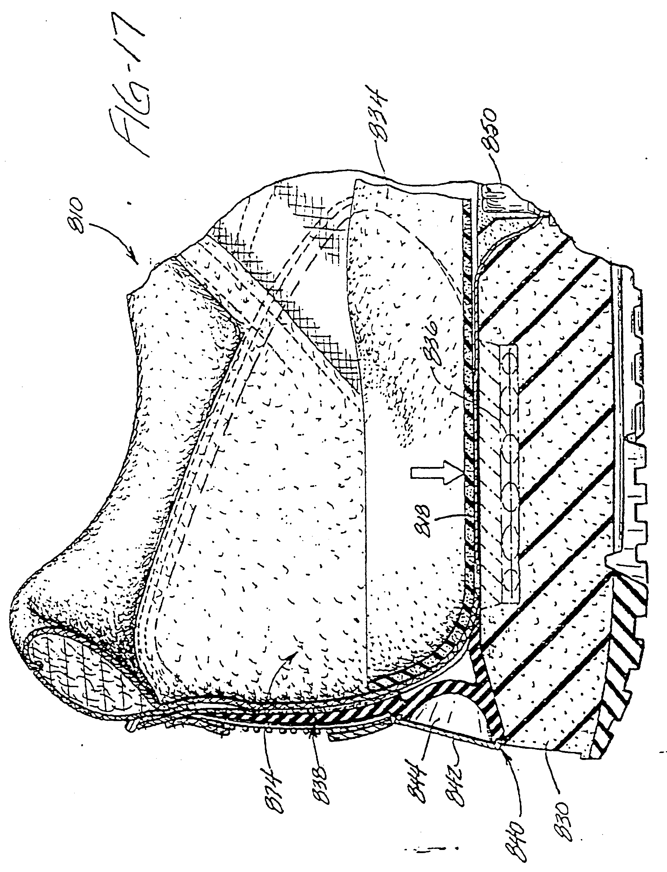

Positioning of components within this preferred shoe can be accomplished in any suitable manner. Referring to **Figures 15** and **16**, a piezoelectric impact sensor **818** is located in the proximal end **830** of the heel and covered by the heel cushion or liner **834**. The sensor **818** is electrically connected to an electronics capsule **850** located in the midsole **812**. The electronics capsule **850** may be mounted in any orientation in addition to the orientation shown. This electronics capsule contains the circuitry and battery pack required to process the signal generated by impacts on sensor **818**. When a signal is generated by sensor **818** of sufficient magnitude to be detected by the circuitry of the capsule **850**, this circuitry turns on LED **826** which is electrically connected to the capsule **850** via leads **828**.

Referring to Figure **16A**, a piezoelectric impact sensor **818A** is located in the proximal end **830** of the heel. This impact sensor **818A** is covered first by a patch **833** which is subsequently covered by the heel portion or liner **834**. The sensor **818A** is electrically connected to an electronic capsule **850** located in the midsole **812**. The electronics capsule **850** may be mounted in any orientation in addition to the orientation shown. This electronics capsule contains the circuitry and battery pack required to process the signal generated by impacts on sensor **818A**. When a signal is generated by sensor **818A** of a sufficient magnitude to be detected by the circuitry of the capsule **850**, this circuitry turns on LED **826** which is electrically connected to the capsule **850** via leads **828A**. The electronic capsule 850 may be insert molded to include the battery, circuit board and electronic components to provide mechanical and environmental integrity to the capsule **850**.

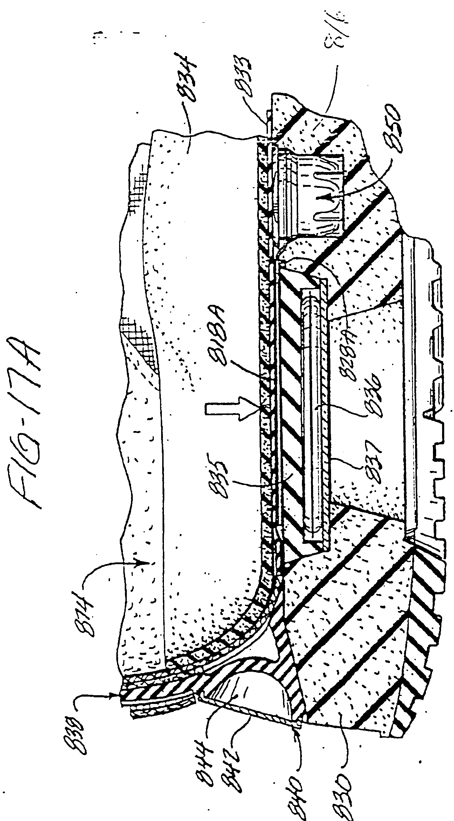

Referring to **Figure 17**, the piezoelectric impact sensor **818** may be located over a cushioning pad **836,** for example, an encapsulated silicon gel cushioning pad, in the heel **838**. This cushioning pad may include a plurality of partitions for directing the semi-liquid from one portion of the cushion to another.

Still referring to **Figure 17**, locating the piezoelectric impact sensor **818** over the cushioning pad **836** enhances the magnitude of the signal generated by sensor **818**. In this embodiment, when an impact force is applied to the impact sensor **818** as indicated by the downward arrow, the impact sensor **818** is further distorted as the cushioning pad **836** gives way beneath it. Since the magnitude of the signal generated by the piezoelectric impact sensor **818** is directly related to the amount of distortion applied to this sensor, the "give" of the cushioning pad permits the sensor **818** to have a greater distortion under the same force than it would if the sensor **818** had been mounted on a more rigid surface. Thus, for the same force, since the distortion with the cushioning pad present is greater, the signal generated by the sensor **818** will be greater.

**Figure 17A** illustrates another embodiment wherein the piezoelectric impact sensor **818A** is located over the cushioning pad 836 in order to enhance the magnitude of the signal generated by sensor **818A**. When an impact force is applied to the impact sensor **818A** as indicated by the downward arrow, the impact sensor **818A** is further distorted as the cushioning pad **836** gives way beneath it. Since the magnitude of the signal generated by the piezoelectric impact sensor **836A** is directly related to the amount of distortion applied to the sensor, the "give" of the cushioning pad permits the sensor **818A** to have a greater distortion under the same force then it would if the sensor **818A** had been mounted on a more rigid surface. Thus, for the same force, since the distortion with the cushioning pad present is greater, the signal generated by the sensor **818A** will be greater. The signal generated by sensor **818A** is transmitted via leads **828A** to the electronics capsule **850.** In **Figure 17A**, the electronics capsule is shown with its base mounted flush with the upper surface of the sole **816**. This base and the associated impact sensor **818A** is covered by patch **833**. Patch **833** is subsequently covered by heel portion **834**.

Still referring to **Figure 17A**, the piezoelectric impact sensor **818A** is located over the cushioning pad **836** which may be an encapsulated silicone gel cushioning pad positioned in the heel **838**. This cushioning pad may include a plurality of partitions for directing the semi-liquid from one portion of the cushion to another. This cushioning pad is typically covered with a GEL cap **835.** A window **837** structure is typically located beneath the gel shock absorber **836**.

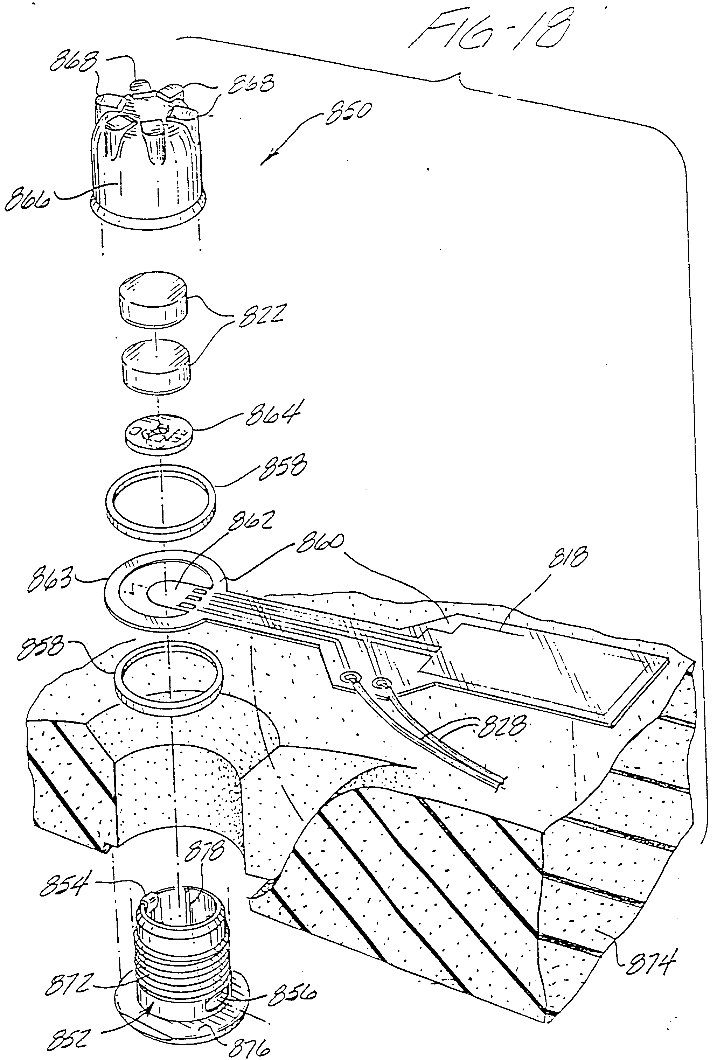

**Figure 18** is a partial exploded view of a preferred embodiment of the present invention showing the electronics capsule **850**, the piezoelectric impact sensor **818** and the LED leads **828** as used in shoe **810** of **Figure 16.** The electronics capsule **850** may be mounted in any orientation in addition to the orientation shown. In this embodiment, a sheet of piezoelectric film is joined to a non-conductive flexible sheet **860**. Conductive leads are deposited on this flexible sheet **860** and electrically connect the signal output from sensor **818** to circuit board **864**. Conductive leads are also deposited on the flexible sheet **860** to electrically connect the outputs from circuit board **864** to LED leads **828**. The conductive leads could also be printed on the flexible sheet (not shown).

**Figure 18A** is a partial exploded view of another preferred embodiment of the present invention showing the electronics capsule **850**, the piezoelectric impact sensor **818A** and the LED leads **828A** as used in shoe **810** of **Figure 16A**. Electronics capsule **850** may be mounted in any orientation in addition to the orientation shown. In this embodiment, a sheet of piezoelectric film is joined to a nonconductive flexible sheet **860A**. Conductive leads are deposited or printed on top and bottom surfaces of this flexible sheet **860A** and electrically connect the signal output from sensor **818A** to circuit board **864**. Conductive leads are also deposited or printed on the flexible sheet **860A** to electrically connect the outputs from circuit board **864** via LED leads **828A** to the LED **826** of **Figure 16A**. The electronics capsule **850** as shown in this embodiment is mounted with its flat lip **876** of the base **852** flush with the upper surface of the sole of the shoe.

The circuitry involved has been reduced in size so that the circuit and LED can be operated for extended periods of time with only two batteries in series. In one test, 790,000 flashes of the LED were achieved using only standard watch batteries.

Additionally, the circuitry, as shown for example in **Figure 12**, is designed so that when the LED is not lit or flashing, the current, i.e., power consumption, is insignificant. Such a low power consumption avoids the necessity of including an ON-OFF switch in the circuit in order to extend battery life.

In an alternative configuration, a solar cell or solar cell array and/or a rechargeable battery could be substituted for the battery.

Referring to **Figure 18** or **18A**, the electronics capsule **850** forms a tightly sealable capsule into which the tongue **862** of the flexible sheet **860** or **860A**, circuit board **864**, and one or more batteries **822** fit. The base of the capsule **852** has a slot **856** through which the tongue **862** intrudes in order to reach the interior of the capsule **850** enabling the circuit board **864** to make electrical contact with the conductive leads deposited on the flexible sheet **860** or **860A**. The electronics capsule **850** may be mounted in any orientation in addition to the orientation shown.

The threads **872** on base **852** mate with corresponding threads (not shown in Figure 18) on cap **866**, which in conjunction with sealing rings **858** enable the capsule **850** to be tightly sealed. This seal can be a waterproof seal. Optionally, the entire capsule can be encapsulated in, for example a polymer, to ensure a waterproof seal and to prevent tampering with the circuitry contained therein.

Included within the electronics capsule **850** is a contact spring **854** mounted on the base **852**. This contact spring **854** electrically connects the outer case of the batteries **822** to the circuit board **864**.

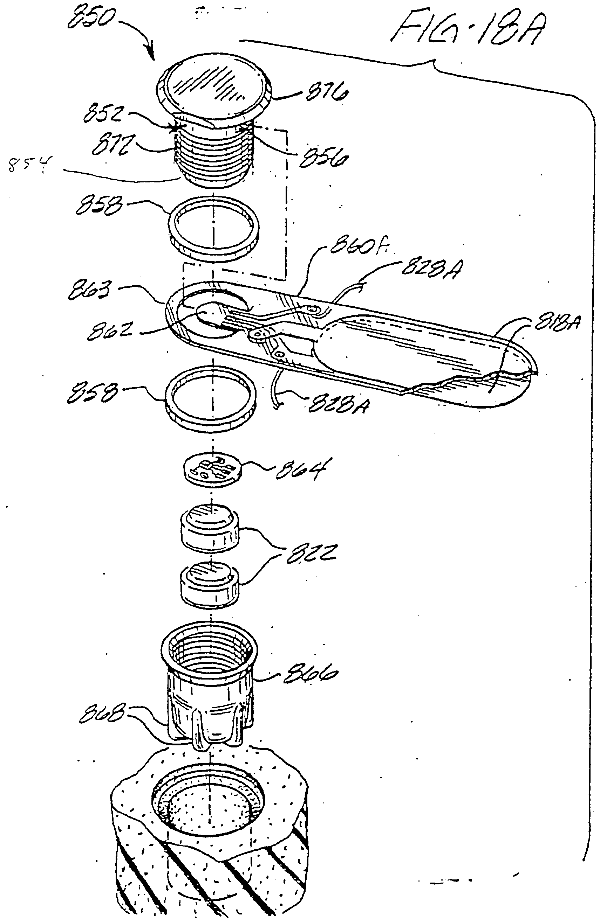

A cross-sectional view of an assembled electronics capsule **850** is shown in **Figure 19**. The electronics capsule **850**, when assembled, comprises a lower sealing ring **858** which sits on the lip **876** of base **852**; the ring **863** of flexible sheet **860** (or **860A**, not shown) fits over base **852** and contacts the upper surface of the lower sealing ring **858**; tongue **862** fits through slot **856**; upper sealing ring **858** is placed over the base **826** and contacts the upper surface of ring **863**; a circuit board **864** and one or more batteries **822** are placed in the center cavity **878** of the base **852**; a contact spring **854** fits into a slot formed in the side of the base **852**, and cap **866** is screwed onto and threadably mates with base threads **872**.

Grips **868** are formed in the cap **866** to enable the cap to be grasped and unthreaded for access to the internal components. Thus, the batteries, circuit board or flexible sheet and impact sensor can be readily replaced.

The capsule is sealed by the compression of the upper and lower sealing rings 858 between the base **852**, the ring **863** of the flexible sheet **860** (or **860A**, not shown), and the cap **866**. The base thread **872** mates with the cap thread **870**. The length of these threads are designed to ensure that the proper pressure is applied to ring **863** and sealing rings **858** to guard against moisture, sweat, bacteria, dust and/or dirt from seeping onto the electronics capsule **850**.

Typically, two batteries **822** are used in series to provide power to operate the circuitry and the light emitting device or devices. The outer case, negative electrode **823** of the upper battery is electrically connected by physical contact with contact spring **854** to the circuit board **864**. The positive electrode **824** of the upper battery is electrically and physically in contact with the negative electrode **823** of the lower battery **822**. The positive electrode **824** of the lower battery **822** is physically and electrically in contact with circuit board **864**.

Conductive leads **862L** deposited or printed on the tongue **862** and the flexible sheet **860** (or **860A**, not shown) electrically contact circuit board **864** and thus connect the piezoelectric impact sensor **818** (or **818A**, not shown) and the LED leads **828** (or **828A**, not shown) and LED **826** to the circuitry located on the circuit board **864**. The circuit board **864** may be keyed to ensure that the proper electrical connections are made.

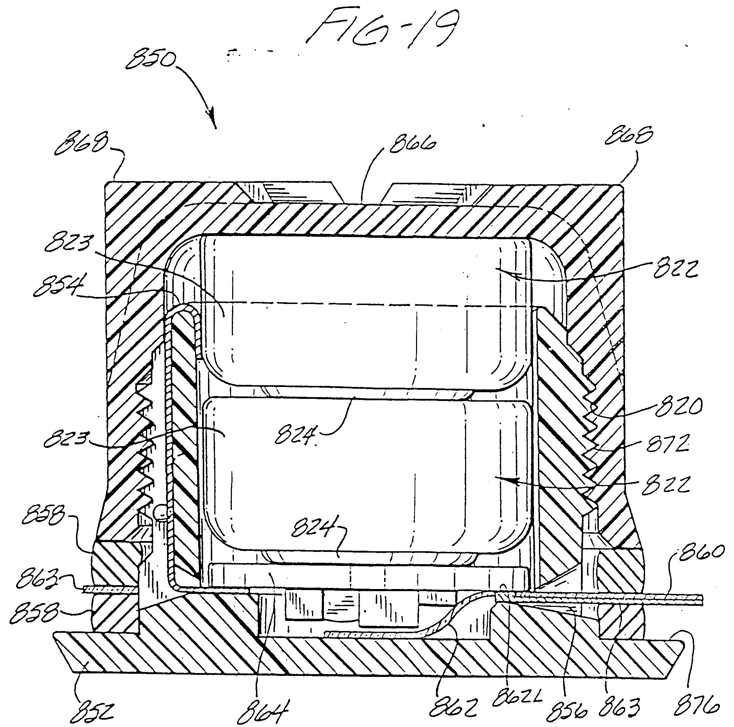

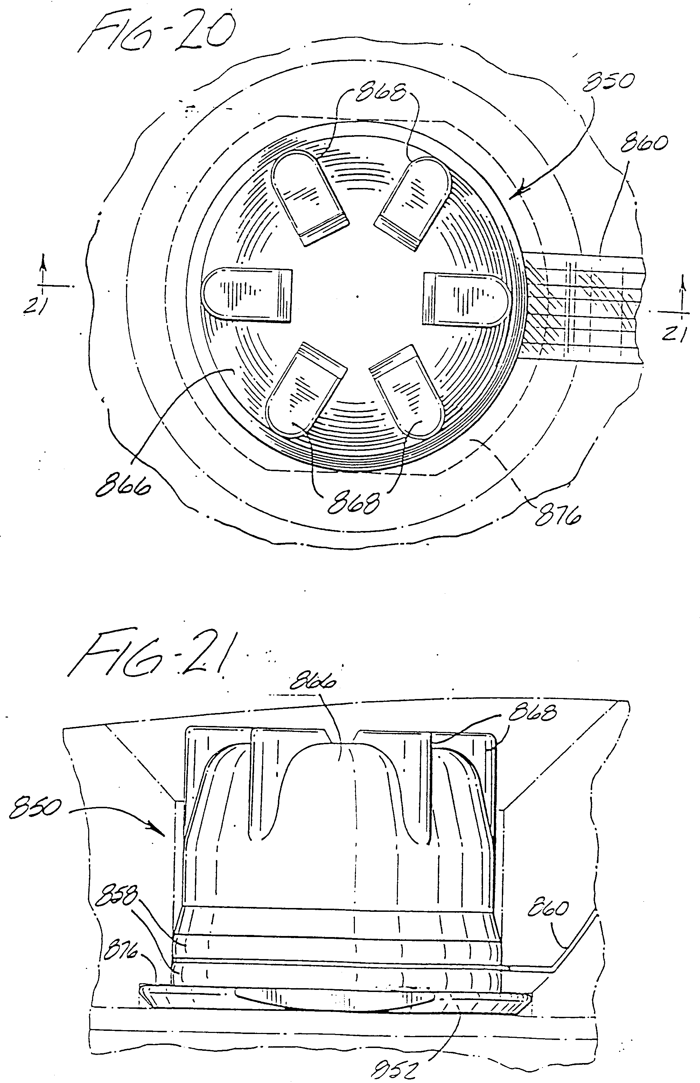

**Figure 20** is a top plan view of the cap **866** of the electronics capsule **850**. **Figure 21** shows a side plan view of the electronics capsule **850** with the cap **866** threaded onto the base **852** and sealing rings **858** sealing the flexible sheet **860** therebetween.

The curved surface of the cap **866** with flat topped grips **868** is designed to maximize the strength of the capsule so that sudden impacts by the wearer of the shoe will not crack, shatter or damage the capsule nor cause the capsule to rise or protrude from its cavity. In addition, the plurality of grips spaced equidistantly about the top circumference of the cap, facilitate easy unthreading of the cap so that the internal components can be replaced and/or repaired.

**Figure 22** diagramically illustrates the critical angles involved in positioning LED **826** in the light chamber **844** of the light unit **840** as shown in **Figures 16** and**17**. In this embodiment, the LED **826** is positioned to maximize the light energy that falls on the lens **842** so as to maximize the light or the image projected to the rear of the shoe.

Considerable effort was devoted to identifying the best angle for placement of an LED in a light chamber in order to maximize the light transmitted from the lens while fitting this light chamber and LED into the heel section (heel counter) of a shoe without adding excessive and obtrusive bulk. **Figures 22, 23,** and **24** reflect a solution to this problem.

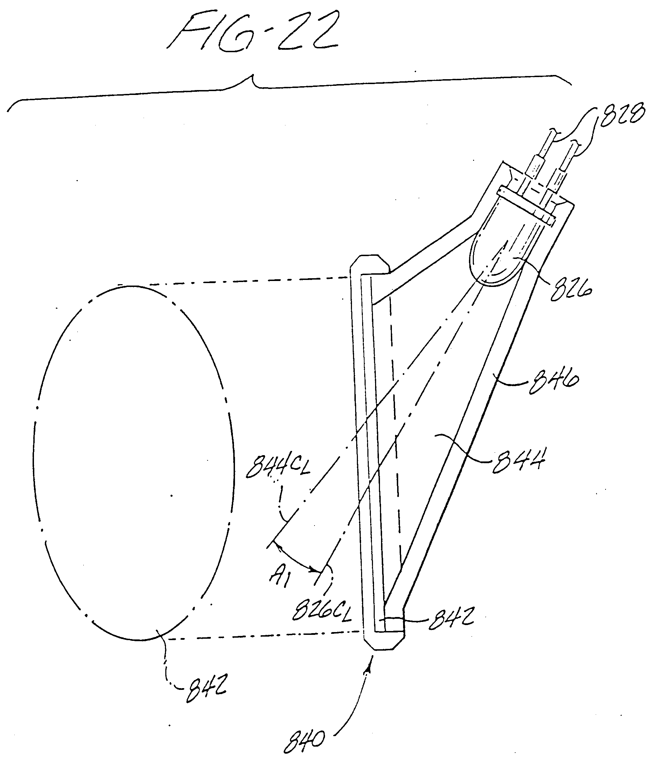

**Figure 23** is a partial cutaway and phantom view of a section of the heel of the shoe of **Figure 15** showing the angular and spatial location of LED **826** and LED leads **828** with respect to the light chamber **844** and the lens **842**.

**Figure 24** illustrates a cross-sectional side view through the heel of **Figure 23**. This side view further shows the placement of LED **826** with respect to light chamber **844** and lens **842**.

The design of the light chamber provides for uniform illumination of lens **842** by LED **826**, without the LED **826** being mounted directly behind, i.e., perpendicular to, the lens **842**. As is apparent from **Figure 22**, the LED center line **826C**_{**L**} is offset by an angle A1 from the light chamber center line **844C**_{**L**}. In one embodiment, these center lines in plan view are offset 4mm to achieve maximum illumination of lens **842** while minimizing the bulk of the light chamber in the heel counter of a shoe.

Referring to **Figure 24**, minimizing the center line **848** offset relative to the datum line **849** of the heel counter is a critical factor in minimizing the bulk of the heel counter **839**. An angle A2 of about 15 degrees minimizes the bulk of the heel counter **839** when light chamber **844** is incorporated into the shoe. Placing the light chamber higher up the heel **838** results in external heel moldings that are larger than necessary, an unacceptable angle for lens **842** in order to provide acceptable rearward visibility and a light chamber that protrudes excessively from the heel.

In another embodiment of the invention, referring to **Figure 2**, a ball **930** is molded in a translucent synthetic rubber material around a central cavity **932** containing a battery-powered light emitting unit **934**. The unit **934** comprises a piezoelectric impact sensor **936**, preferably of PVDF similar to the piezoelectric impact sensor **218** of **Figure 1**. The piezoelectric impact sensor **936** is electrically connected to a ball circuit similar to the circuit **220** of **Figure 3**. The ball circuit is powered by a battery pack **940** similar to the battery pack **222** of **Figure 3.** The difference is that the ball circuit turns on two LEDs **942** both similar to the LED **226** of **Figure 1**. The LEDs **942** are positioned so as to emit light outwards from the central cavity **932** in generally opposite directions.

In operation, each time the ball **930** impacts a hard surface, e.g. each time it is bounced, the piezoelectric impact sensor **936** is stressed, and so produces a pulse of electrical energy which triggers a monostable circuit that allows both LEDs **942** to light for a predetermined time period. Since the LEDs **942** emit light in generally opposite directions, one or the other LED tends to be visible regardless of the direction from which the ball **930** is viewed.

The fact that the ball **930** is molded in a translucent (as opposed to a fully transparent) material means that the emitted light tends to be diffused, and the components making up the light emitting unit **934** (i.e. the piezoelectric impact sensor **936**, the ball circuit, the battery pack **940** and the LEDs **942**) are not clearly and individually visible as separate components.

It should be noted that the LED **326** of the shoes of the present invention or the LEDs **942** of the ball of the present invention can be replaced by sound-emitting devices. Similarly, the circuitry can be modified so that both the LED and the sound-emitting device can be incorporated in the same product.

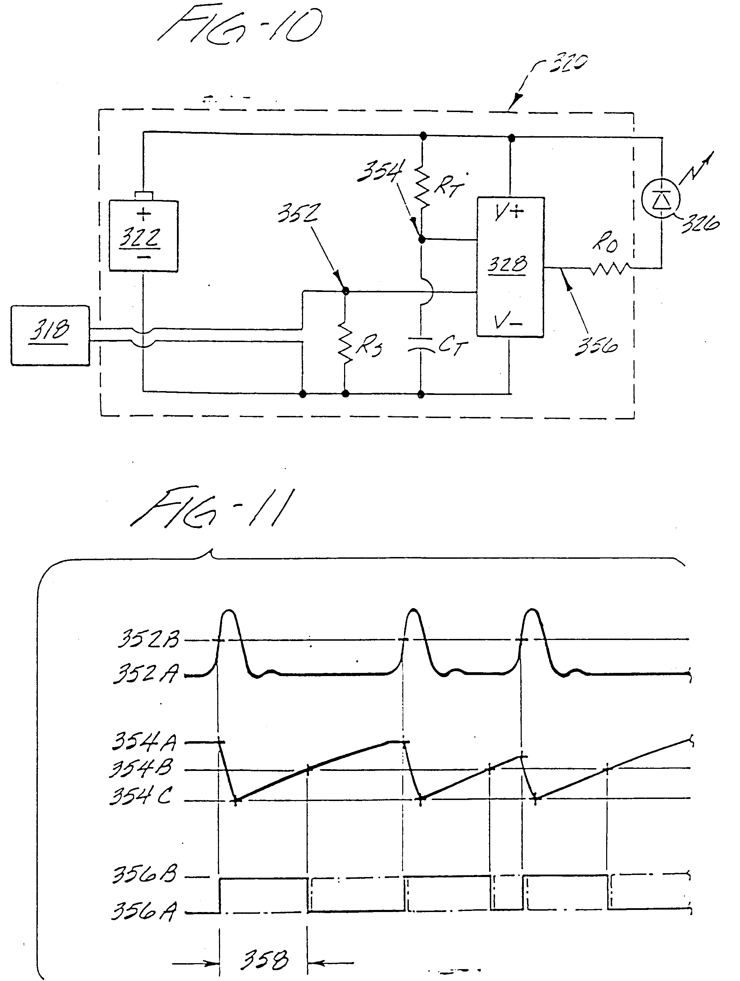

Referring to **Figure 9**, it is seen that the present invention can also be used in connection with a fishing lure **986**, comprising a piezoelectric impact sensor **918**, a circuit **920**, a battery pack **922** and an LED **926**. The lure **986** can be shaped or molded to resemble a fish having fins **988** and a tail **990**. Additionally, the fishing lure may comprise a fish shape with optical fibers **992** conducting light from the LED **926** to, e.g., the fins and/or tail (or even eyes) of the fish-shaped lure. In operation, impacts from jerks on the fishing line or the like are sensed by the piezoelectric impact sensor **918**, these impacts generate a trigger signal to circuit **920** which in turn lights/flashes the LED **926** for a preselected time period.

It will be understood that various changes in the details, materials, and arrangements of parts may be made by those skilled in the art within the principle and scope of the invention.

## Claims

1. An impact responsive shoe comprising:
impact sensor means comprising a polymeric sheet of a piezoelectric material for generating trigger signals upon impact;
output means for generating an output signal, the output means comprising a light emitting diode (LED);
circuit means comprising a monostable circuit, the circuit means interconnecting the impact sensor means and the output means, the circuit means including power means for powering the output means, the circuit means responding to trigger signals from the impact sensor means to power the output means to deliver an output signal associated with an impact, and further includes
a resistive - capacitive (R-C) combination means for determining the length of time that the light emitting diode is lit.

2. The apparatus of Claim 1 wherein the output means comprises a plurality of LEDs.

3. The apparatus of Claim 1 wherein the impact sensor means comprises a plurality of impact sensors.

4. A shoe that lights in response to impacts comprising:
impact sensor means comprising a polymeric piezoelectric material for generating trigger signals upon impact;
a light emitting diode (LED);
battery means for powering the LED; and
circuit means interconnecting the impact sensor means, the LED and the battery means, the circuit means responding to the trigger signals to interconnect the battery means with the LED to light the LED upon an impact.

5. The apparatus of Claim 4 wherein the LED is positioned within the shoe and optic fibers conduct the light to the outside of the shoe.

6. The apparatus of Claim 5 wherein the optic fibers include a plurality of notches for permitting the emission of light therefrom.

7. The apparatus of Claim 4 wherein the circuit means comprises a monostable multivibrator circuit means comprising:
a sensitivity resistive means across which the trigger signal of the impact sensor means is applied;
a monostable multivibrator circuit;
a first resistive means for limiting the current through the LED;
a second resistive means coupled with a capacitive means to form an R-C timing circuit means; and
means for applying the voltage developed across the capacitive means of the R-C timing circuit means to the input of the monostable multivibrator circuit means.

8. An impact responsive shoe comprising:
impact sensor means comprising a polymeric sheet of a piezoelectric material for generating trigger signals upon impact;
output means for generating an output signal;
battery means for powering the output means; and
circuit means interconnecting the impact sensor means, the output means and the power means, the circuit means responding to the trigger signals to interconnect the power means with the output means to deliver an output signal associated with an impact.

9. The apparatus of Claim 8 wherein the output means comprises an information display device comprising a liquid crystal display (LCD).

10. The apparatus of Claim 8 wherein the output means comprises a light emitting device comprising a gas discharge lamp.

11. The apparatus of Claim 8 wherein the output means comprises a plurality of LEDs.

12. The apparatus of Claim 8 wherein the impact sensor means comprises a plurality of impact sensors.

13. The apparatus of claim 11 wherein the plurality of LED's is arranged sequentially in the upper of the shoe to form a bar graph display of information produced by the impact sensor means and the circuit means.

14. The apparatus of Claim 13 wherein the circuit means comprises a microprocessor means to provide preprogrammed control of the plurality of LEDs.

15. The apparatus of Claim 14 further comprising:
temperature sensor means for sensing temperature within the shoe;
temperature output means for indicating the relative temperature within the shoe; and
temperature circuit means for interconnecting the temperature sensor means with the temperature output means.

16. The apparatus of Claim 15 wherein the plurality of LEDs comprise a bar graph display of the temperature sensed within the shoe.

17. A impact responsive lighted shoe comprising:
impact sensor means comprising a polymeric piezoelectric material for generating trigger signals upon impact;
a light emitting diode (LED);
battery means for powering the LED;
circuit means for interconnecting the impact sensor means, the LED and the battery means; the circuit means responding to the trigger signals to interconnect the battery means with the LED to light the LED upon an impact; and
encapsulation means for encapsulating the battery and circuit means.

18. The apparatus of claim 17 wherein the encapsulation means comprises a sealed capsule comprising:
a base having a cylindrical portion and a lip thereon;
a lower sealing ring sized to receive the cylindrical portion of the base and to coact with the lip;
means for releasably accepting leads from the impact sensor means;
an upper sealing ring sized to receive the cylindrical portion of the base, the upper sealing ring and the lower sealing ring coacting to position and hold leads from the impact sensor means and the LED and to seal the capsule;
circuit board means for supporting the circuit means, the circuit board means sized to be accepted within the cylindrical portion of the base, the circuit board means being in electrical contact with the leads and the battery;
a plurality of batteries sized to be received within the cylindrical portion of the base, the batteries being in electrical contact with the circuit means on the circuit board means;
a contact spring means positionned within the cylindrical portion of the base, one end of the contact spring means contacting the negative terminal of one battery and the other end contacting the circuit means on the circuit board means; and
a cap threadably attachable to the base, the cap and the base coacting to close and seal the capsule.

19. A product adapted to emit light or sound in response to impact, the product comprising:
a battery-powered light or sound emitting unit at least partially molded therein,
the unit comprising an impact sensor made from a polymeric piezoelectric material,
a power source battery,
a light or sound emitting device, and
a circuit connected to the impact sensor and responsive to a trigger signal produced thereby in response to impact to energize the light or sound emitting device from the battery.

20. The apparatus of Claim 19 comprising a light emitting device disposed inside the product and further comprising optical fiber means arranged to conduct the light emitted by the light emitting device to an outside surface of the product.

21. The apparatus of Claim 19 wherein the product is a fishing lure.

22. The apparatus of Claim 20 wherein the product is a fishing lure shaped like a fish with fins and a tail, wherein the optical fiber means comprises a bundle of optical fibers at least some of which conduct light emitted by the light emitting device to the fins and the tail of the fishing lure.

23. The apparatus of Claim 19 wherein the product is a ball.

24. The apparatus of Claim 23, including two light emitting devices both arranged to be energized by the circuit and arranged to emit light in generally opposite directions.

25. The apparatus of Claim 19 wherein the product is a shoe.

26. The apparatus of Claim 25 comprising a plurality of impact sensors distributed through the sole of the shoe and a circuit designed to light a plurality of light emitting devices so as to display the relative Impact sensed by each impact sensor.

27. An impact responsive shoe comprising:
input sensor means for generating input signals;
information display means for displaying information;
battery means for powering the input sensor means, information display means and a microprocessor circuit means;
microprocessor circuit means for accepting input signals generated by the input sensor means, analyzing and processing these signals, and outputting signals to the information display means.

28. An impact responsive shoe comprising:
impact sensor means comprising a polymeric sheet of a piezoelectric material for generating trigger signals upon impact;
an information display means for indicating various exercises performed by a wearer of the shoe;
circuit means for processing the trigger signals from the impact sensor means and for controlling the information displayed by the information display means;
wherein the circuit means comprises microprocessor circuitry and power means for powering the information display device.

29. An impact responsive shoe comprising:
impact sensor means comprising a polymeric sheet of a piezoelectric material for generating trigger signals upon impact;
temperature sensor means for generating signals indicative of the temperature inside the shoe;
circuit means for processing the trigger signals from the impact sensor means and the temperature sensor means and for controlling output means; and
output means for activating visual or audible output devices that indicate the impacts detected and the temperature sensed within the shoe.

30. An impact responsive shoe comprising an impact sensor in the form of a sheet of a piezoelectric material molded into the sole of the shoe, a battery, a plurality of light-emitting devices, and circuit means responsive to electrical energy produced by the piezoelectric material in response to impact to selectively energize the light-emitting devices from the battery in dependence upon the amount of said electrical energy, whereby the light-emitting devices provide a visual indication of the magnitude of the pressure exerted on the sole of the shoe.

31. A shoe as claimed in Claim 30, wherein the light-emitting devices are arranged to form a bar graph display.

32. A shoe comprising temperature sensing means responsive to the temperature within the shoe, a battery, a plurality of light-emitting devices, and circuit means responsive to the temperature sensing means to selectively energize the light-emitting devices from the battery so as to provide a display indicative of the temperature within the shoe.

33. A shoe as claimed in claim 32, wherein the light-emitting devices are arranged as a linear array.

34. A shoe as claimed in claim 32, wherein the circuit means includes a ladder network and a plurality of threshold circuits.

35. A shoe comprising a battery, at least one light-emitting device and circuit means for energizing said light-emitting device from the battery at, at least, one programmable rate providing a pace-setting facility to the wearer of the shoe.

36. A shoe as claimed in Claim 35, wherein the shoe further includes a piezoelectric impact sensor for producing electrical signals to each impact of the shoe, the circuit means being responsive to said electrical signals to modify said programmable rate or rates.

37. A shoe as claimed in Claim 36, wherein said circuit means includes a microprocessor.
